# EUROPEAN PATENT SPECIFICATION

(11) **EP 3 750 878 B1**
(45) Date of publication and mention of the grant of the patent: **14.05.2025**
(21) Application number: 19180240.4
(22) Date of filing: 14.06.2019
(51) Int. Cl.: C07D 231/12, C07D 235/18, C07D 207/337, C07D 249/08, C07D 249/06, C07D 471/04, C07D 261/08, A61K 31/40, A61K 31/405, A61K 31/4164, A61K 31/42, A61K 31/437, A61P 25/28, A61P 29/00, A61P 33/00

(54) **HETEROAROMATIC INHIBITORS OF ASTACIN PROTEINASES**
HETEROAROMATISCHE INHIBITOREN VON ASTACINPROTEINASEN
INHIBITEURS HÉTÉROAROMATIQUES DE PROTÉINASES ASTACINES

(43) Date of publication of application: 16.12.2020
(73) Proprietor: Vivoryon Therapeutics N.V., 06120 Halle (DE)
(72) Inventor: Ramsbeck, Daniel, 06118 Halle (Saale) (DE); Tan, Kathrin, 06122 Halle/Saale (DE); Schlenzig, Dagmar, 06114 Halle/Saale (DE); Buchholz, Mirko, 06114 Halle (Saale) (DE); Cynis, Holger, 06120 Halle (Saale) (DE); Schilling, Stephan, 06130 Halle (Saale) (DE)
(74) Representative: Maikowski & Ninnemann Patentanwälte Partnerschaft mbB

(56) References cited:
- WO-A1-2006/114263
- WO-A1-2017/182433
- US-A- 4 146 721
- E. ADIGUZEL ET. AL.: "Synthesis and characterization of two new hydroxamidc acid derivatives and their metal complexes. An investigation on the keto/enol E/Z and hydroxamate/hydroximate forms.", JOURNAL OF MOLECULAR STRUCTURE, vol. 1127, 19 July 2016 (2016-07-19), pages 403 - 412, XP002793302

## Description

### FIELD OF THE INVENTION

The present invention relates to novel hydroxamic acid derivatives useful as inhibitors of astacin metalloproteinases, in particular procollagen C-proteinase (PCP) enzymes, meprins, ovastacin and/or nematode astacins; more particularly human or mammalian meprin α, meprin β, BMP-1, ovastacin and/or DPY-31 from nematodes; pharmaceutical compositions comprising such compounds; methods for treatment or prophylaxis of diseases or conditions, especially such that are related to said metalloproteinases; and compounds and pharmaceutical compositions for use in such methods.

### BACKGROUND ART

Proteinases from the astacin family represent a widespread group of metalloproteinases occurring in lower as well as in higher organisms. The astacins are a subfamily of the metzincin superfamily of proteinases, all of which share the conserved zinc binding sequence in their active site, a conserved methionine-containing turn (Met-turn) backing the zinc site and strikingly similar three-dimensional structures of their catalytic domains (Sterchi et al., Mol Aspects Med. (2008), 29(5): 309-328). In the human organism, there are at least four enzymes of the astacin family: the bone morphogenetic protein 1 (BMP-1), the two meprins (meprin α and meprin β), as well as ovastacin. Additionally, nematode parasites such as trichina and hookworms secrete a variety of astacins which are found throughout the entire taxon *Nemathelminthes*.

BMP-1 and the meprins participate in collagen formation and thus play a role in various diseases associated with pathological formation of connective tissue, such as fibrotic conditions including pulmonary fibrosis and keloids. Furthermore, meprins have been described as being associated with various types of cancer, Morbus Alzheimer, acute renal failure and chronic inflammatory bowel diseases. Meprin inhibitors, therefore, represent novel candidates for the treatment of such diseases.

In turn, ovastacin plays a role in reproduction. Upon fertilization of the oocyte, hardening of the zona pellucida (ZPH) takes place which is induced by the ovastacin activity and provides mechanical protection to the fertilized egg by establishing a block against polyspermy and ensuring normal development of the embryo. However, if such hardening occurs too early, or even in the absence of fertilization, infertility can arise. Inhibition of ovastacin can therefore represent a novel approach for addressing an unfulfilled desire to have a child and/or in the context of *in vitro* fertilization and reproductive medicine.

Furthermore, multiple astacin proteases have been identified in nematodes. These play an important role in the formation of the collagen-containing cuticula. Nematode infections represent a major issue, in particular in livestock farming. Furthermore, nematode parasites are responsible for multiple tropical diseases. Inhibition of these enzymes interferes with the formation of an external protective layer and leads to mitigated growth or death of the worms. Inhibitors of such nematode astacins, therefore, represent novel anthelmintic agents for the treatment of parasite infections.

### Metzincins and the Astacin Family

As noted above, the astacins are a family of multi-domain metallopeptidases with manifold functions in the metabolism. They are either secreted or membrane-anchored and are regulated by being synthesized as inactive zymogens and by co-localizing protein inhibitors. The distinct family members consist of N-terminal signal peptides and pro-segments, zinc-dependent catalytic domains, further downstream extracellular domains, transmembrane anchors, and cytosolic domains. The catalytic domains of four astacins and the zymogen of one of these have been structurally characterized and shown to comprise compact ~200-residue zinc-dependent moieties divided into an N-terminal and a C-terminal sub-domain by an active-site cleft. Astacins include an extended zinc-binding motif (HEXXHXXGXXH) which includes three metal ligands and groups them into the metzincin clan of metallopeptidases. In mature, unbound astacins, a conserved tyrosine acts as an additional zinc ligand, which is swung out upon substrate or inhibitor binding in a 'tyrosine switch' motion. Other characteristic structural elements of astacin catalytic domains are three large α-helices and a five-stranded β-sheet, as well as two or three disulfide bonds. The N-terminal pro-segments are variable in length and rather unstructured. They inhibit the catalytic zinc following an 'aspartate-switch' mechanism mediated by an aspartate embedded in a conserved motif (FXGD). Removal of the pro-segment uncovers a deep and extended active-site cleft, which in general shows preference for aspartate residues in the specificity pocket (S₁') (Gomis-Rüth et al., Biol. Chem. (2012), 393: 1027-1041).

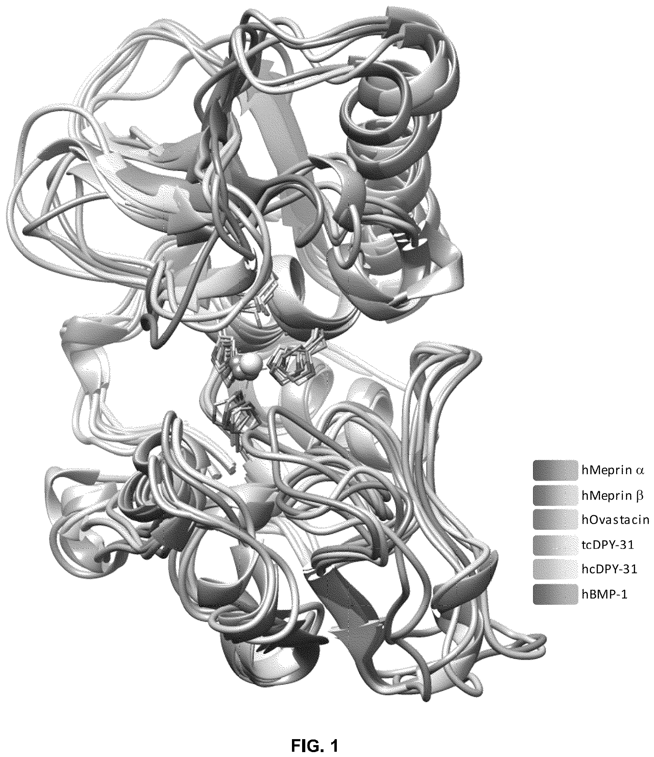

Besides the family of astacins, further metalloproteinase families within the metzincin superfamily include ADAMs ("A Disintegrin And Metalloproteinase", such as ADAM metallopeptidase domain 17 (ADAM17), also called TACE (tumor necrosis factor-α-converting enzyme); and ADAM10, i.e. α secretase) and MMPs ("Matrix Metalloproteinases", such as MMP1 collagenase; MMP2 gelatinase; MMP9; and MMP13), as shown in Fig. 1 of Sterchi et al., Mol Aspects Med. (2008), 29(5): 309-328.

For instance, TACE (ADAM17) inhibitors are known. However, many of these compounds display non-selectivity, being potent inhibitors of matrix metalloproteases, and in particular MMP-1, inhibition of which has been postulated to cause joint pain in clinical trials of metalloproteases inhibitors, as described in WO 2008/142376 A1. Therein, bicyclosulfonyl acid (BCSA) compounds are disclosed, all of which share as a common structural motif 2-(1,1-dioxo-2-phenyl-2,3-dihvdro-1H-benzo[d]isothiazol-3-yl)acetic acid, (1,1-dioxo-2-phenyl-2,3-dihydro-1H-isothiazolo[4,5-b]pyridin-3-yl)acetic acid, 2-(1,1-Dioxo-2-phenyl-2,3-dihydro-1H-benzo[b]thiophen-3-yl)acetic acid or substituted derivatives thereof. In other words, all the compounds therein have a SO₂ group at the 1-position within the 5-membered ring of the bicyclic system. Furthermore, all of the 5-membered rings forming part of the bicyclic systems described therein a partially saturated, i.e. puckered. Finally, the compounds disclosed therein are inhibitors of TACE (an ADAM metalloprotease) rather than astacin metalloproteinases.

In the human and mouse genomes, genes encoding astacin proteases include *BMP-1, tll1, tll2, mep1a, mep1b,* and *astl.* The first three code for the so-called tolloid subgroup, which includes the protein BMP-1 and its major splice variant, mammalian tolloid. These two are also known as procollagen C-proteases and are important for extracellular matrix assembly. Genes *mep1a* and *mep1b* encode the multi-domain proteins meprin α and meprin β, respectively. A further subgroup of astacins in vertebrates comprises the so-called hatching enzymes, represented by just one member in mammals termed ovastacin which plays a role in sperm-egg interaction. Finally, the genomes of lower invertebrates, and in particular nematodes, contain more astacin genes than mammalian genomes (up to about 40 in nematodes such as *Caenorhabditis elegans* (Gomis-Rüth et al., Biol. Chem. (2012), 393: 1027-1041).

### Meprins

Specifically, meprin α and β both represent zinc-dependent metalloproteases of the astacin family and the metzincin superfamily. They show a similar domain structure and the human enzymes are of 45% sequence homology to each other. Meprin β is a type 1 transmembrane protein with extracellular protease activity whereas meprin a is shed during the secretory pathway and secreted into extracellular space. Both enzymes are expressed as zymogens with high expression rates in epithelial cells of the kidney and intestine, and they have been demonstrated in intestinal leukocytes, skin and certain cancer cells.

The meprins show distinct substrate specificity with a preference of acidic amino acids in the P1'-position (Becker-Pauly et al. Mol.Cell Proteomics (2011), doi: 10.1074/mcp.M111.009233). Several *in vitro* substrates have been identified, including extracellular matrix proteins, peptide hormones and cytokines. Known *in vitro* substrates of meprin β comprise orcokinin, gastrin 17, Peptide YY, kinetensin, osteopontin, interleukin 1β, APP, MUC 2 mucin, and cystic fibrosis transmembrane conductance regulator E-cadherin, whereas known *in vitro* substrates of meprin α comprise bombesin, neurotensin, Substance P, angiotensin I, luteinizing hormone releasing hormone, valosin, vasoactive intestinal peptide, bradykinin, α-melanocyte stimulating hormone, MCP-1, and occludin. Known *in vitro* substrates of both meprin β and α are, e.g., the Gastrin-releasing peptide, and Cholecystokinin. Although the function of meprins *in vivo* still remains to be elucidated, there is increasing evidence for their role in collagen assembly, inflammation, intestinal immune response and neurodegeneration.

The lack of meprin β and α in mouse or the use of Actinonin (a meprin inhibitor) have been shown to protect against renal injury and bladder inflammation (Bylander et al., American journal of physiology. Renal physiology (2008), 294(3): F480-90; Yura et al., American journal of physiology. Renal physiology (2009), 296(1): F135-44). Accordingly, meprin β and α appear to be involved in the pathogenesis and/or disease progression of, e.g., nephritis, renal injury, renal ischemic injury, ischemic acute tubular necrosis, acute renal failure, and bladder inflammation.

Both enzymes have been demonstrated to be C- and N-procollagen proteinases and to induce collagen maturation and assembly (Biasin et al., The Journal of pathology (2014), 233(1): 7-17; Prox et al., Matrix biology (2015), 44-46: 7-13). Under fibrotic conditions (keloids, pulmonary hypertension), overexpression of the enzymes has been found in these studies. Accordingly, meprin β and α appear be involved in the pathogenesis and/or disease progression of, e.g., fibrosis and fibrotic conditions (keloids, pulmonary hypertension) and interstitial lung disease (ILD).

Meprin β has been shown to act as β-secretase of amyloid precursor protein to form amyloid β (Aβ) peptides *in vitro* (Bien et al., The Journal of biological chemistry (2012), 287(40): 33304-33313). The Aβ peptide, which is abundantly found in the brains of patients suffering from Alzheimer's disease, is central in the pathogenesis of this disease. Said study showed that, in contrast to BACE I, meprin β is capable of formation of N- terminally truncated Aβ and therefore might be involved in the generation of potentially more toxic species of Aβ. Accordingly, meprin β appears to be involved in the pathogenesis and/or disease progression of, e.g., Alzheimer's disease.

Meprin α has been shown to be a susceptibility gene for IBD (Crohn's disease, ulcerative colitis) and that its absence increases chronic inflammation, while meprin β has pro-inflammatory activity and its lack results in some protection from injury (Banerjee et al., Am. J. Physiol. Gastrointest. Liver Physiol. (2011), 300(2): G273-82). Accordingly, meprin β and α appear to be involved in the pathogenesis and/or disease progression of, e.g., chronic inflammation, Crohn's disease, ulcerative colitis, and inflammatory bowel disease (IBD). Pro-angiogenetic activity and non-polarized secretion have been described for meprin α, thereby increasing invasiveness of colorectal cancer (Lottaz et al., PloS one (2011), 6(11): e26450). Accordingly, meprin α is relevant to the pathogenesis and/or disease progression of cancer, especially colorectal cancer.

Several broad-spectrum metalloproteases and MMP inhibitors have been elucidated concerning their inhibitory activity towards meprin α and β (Broder et al., The Biochemical Journal (2013), 450: 253-264). Although some compounds showed inhibition of meprin α, for all the compounds, the inhibition of Meprin β was much lower (exhibiting inhibition constants in the micromolar range) or were lacking acceptable drug-like properties (Madoux et al., Biopolymers (2014), 102(5): 396-406). A phosphinic inhibitor of meprin β (PMI) is described in Broder C., Characterization of the metalloproteases meprin α and meprin β within the protease web (August 2013; Doctoral dissertation; Universitätsbibliothek Kiel; Accession No. urn:nbn:de:gbv:8-diss-146034; pp. 29, 53).

WO 2017/182433 A1 describes hydroxamic acid derivatives as inhibitors of meprin β and/or a, pharmaceutical compositions comprising such compounds, methods for treatment or prophylaxis of diseases or conditions, especially such that are related to meprin β and/or α, and compounds and pharmaceutical compositions for use in such methods. However, therein are described compounds that are secondary or tertiary amine derivatives of w-aminoalkylhydroxamic acids (such as secondary or tertiary amine derivatives of 2-aminoethanehydroxamic acid or 3-aminopropanehydroxamic acid). Ramsbeck, D. et al., Bioorganic & Medicinal Chemistry Letters 2017, 27(11), 2428-2431 describe inhibitors of meprin β. However, the compounds described therein are based on a sulfonamide scaffold.

### PCP Enzymes; BMP-1

The bone morphogenetic protein BMP-1 belongs to the procollagen C-proteinase (PCP) enzymes, which are a small group of closely-related zinc metalloproteinases with the ability to specifically cleave the carboxyl pro-domains of fibrillar collagens (Turtle et al., Expert Opin., Ther. Patents (2004), 14(8): 1185-1197). The PCPs are part of the astacin family of zinc metalloproteinases. Astacin, a digestive enzyme from crayfish, is one of the smallest members in this family of diverse proteases. BMP-1 has a 39% sequence homology with astacin, and, like all members of this family contains a conserved zinc-binding motif, HEXXHXXGXXH. The astacin active-site domain is contained within a cleft between large N- and C-terminal domains. The active-site zinc is coordinated by the three histidine residues of the consensus sequence, a tyrosine sequence and a water molecule. The glutamic acid of the consensus sequence acts as a general base on the zinc bound water molecule, the nucleophile attacking the scissile amide bond of the substrate.

BMP-1 is the smallest of the PCP isoforms. In addition to BMP-1, four closely related mammalian PCPs have been discovered: mTLD, TLL-1 and -2 and BMP-1/His enzymes. All five enzymes share a high sequence homology in the catalytic astacin-like domain, and also appear to have redundancy in some functions. The PCPs have been primarily noted for their procollagen possessing ability, a process required for fibrosis and wound healing, and their relation to conditions that promote collagen remodeling. The concept of PCP inhibition as an antifibrotic approach is based on the presumption that blockade of PCP activity does not in itself reduce formation of procollagen but prevents the formation of highly structured collagen fibrils. Without cleaving by PCP, procollagen remains soluble to 0.5-1.0 mg/ml and is not as readily incorporated into collagen fibrils. Inhibition of PCP has been associated with the deposition of collagen which is more susceptible to rapid degradation by matrix metalloproteinases (MMP). Therefore, inhibition of PCP enzymes, and in particular BMP-1, has been established as a therapeutic approach for the treatment of fibrosis/scarring as well as diseases and conditions associated therewith (Turtle et al., Expert Opin. Ther. Patents (2004), 14(8): 1185-1197).

Scar formation is part of the natural healing response to tissue or organ damage. The wound healing process consists of blood coagulation, inflammatory response, tissue formation, and tissue remodeling, with the remodeled tissue becoming the scar or fibrotic tissue. Under ideal wound healing conditions, the fibrotic response produces minimal scar tissue, leaving most of the functional tissue intact, thereby preserving organ function. Fibrotic diseases are characterized by fibroblast over-proliferation and excessive deposition of collagen, which presents itself as dense fibers running through the tissue. The resulting fibrotic tissue blocks arteries, immobilizes joints and stiffens internal organs, obstructing the body's ability to maintain normal functions. Fibrotic diseases remain the number one killer in the world, accounting for more than 45% of the entire mortality in the United States, but there are currently no adequate therapies for most fibrotic conditions (Turtle et al., Expert Opin. Ther. Patents (2004), 14(8): 1185-1197).

Although, fibrosis is usually not a primary pathological event but follows trauma, infection, inflammation or surgical procedures, it may occur for unknown reasons, and may also have genetic and autoimmune components. Therefore, fibrosis can occur in any organ and accompanies many disease states, such as hepatitis (liver cirrhosis), hypertension and myocardial infarction (heart failure), asthma and pulmonary hypertension (pulmonary fibrosis), scleroderma (fibrotic skin and internal organs), diabetes (nephropathy), atherosclerosis (fibrotic blood vessels). In addition, hypertrophic dermal scarring and keloids can result in disability and disfigurement, and acute CNS scarring following traumatic injuries, such as strokes and spinal cord injuries, presents a major barrier for neuronal regeneration. Development of obliterative fibrosis of the hollow structures within grafts is the common denominator of the chronic allograft rejection. The process of fibrosis and wound healing, regardless of its etiology, actually recapitulates the major event commonly associated with embryogenesis. However, the underlying mechanisms are quite different; in adults, healing of wounds is often accompanied by scar formation, whilst fetal wounds heal with minimal or no scar formation. In addition to the serious, often life-threatening, chronic fibrotic disorders, acute fibrotic conditions, such as post-surgical scarring and dermal scarring, represent an important potential market for antifibrotics. Millions of surgical procedures are performed annually. Surgical scarring can complicate medical procedures and limit recovery, and the therapeutic need goes far beyond cosmetic applications: fibrosis resulting from gynecological procedures can cause infertility; fibrosis after eye surgery can result in blindness; fibrosis following angioplasty can result in restenosis; and fibrosis following surgery on joints can severely limit range of motion. Finally, PCP inhibitors have also been postulated to be useful in preventing local invasion, recurrence and metastasis of squamous cell carcinoma (SCCs), malignant keratinocytes, which are a common form of cancer, particularly in skin cancers. In summary, inhibitors that are selective for PCPs, such as BMP-1, are thought to be optimal for halting the excess collagen deposition associated with pathological fibrotic conditions and related diseases (Turtle et al., Expert Opin. Ther. Patents (2004), 14(8): 1185-1197).

However, it has also been noted that the non-specific inhibition of MMPs has been associated with muscular skeleton-stiffening side effects seen in human clinical trials (Turtle et al., Expert Opin. Ther. Patents (2004) 14(8): 1185-1197, p. 1190). Therefore, there is a demand for selective PCP, in particular BMP-1 inhibitors, which are preferably selective over MMPs such as MMP2, MMP9, etc.

### Hatching Enzymes; Ovastacin

Inhibition of ovastacin has been reported to be directly related to mammalian gamete fusion and is thus of high relevance to reproductive biology and fertility control (Stöcker et al., Biol. Chem. (2014), 395(10): 1195-1199). The zona pellucida, a glycoprotein matrix surrounding the mammalian oocyte, hardens after intrusion of the first spermatozoon, thus protecting the embryo until implantation and preventing multiple fertilizations (polyspermy). Definitive zona hardening is mediated by the metalloprotease ovastacin, which is released from cortical granules of the oocyte upon sperm penetration. However, traces of ovastacin seep from unfertilized eggs to cause zona hardening even in the absence of sperm. These small amounts of protease are inactivated by the plasma protein fetuin-B, thus keeping eggs fertilizable. Once a sperm has penetrated the egg, ovastacin from cortical vesicles overrides fetuin-B and initiates zona hardening. The molecular mechanism of fertilization control was discovered in the highly specific interaction of fetuin-B and the cortical granule protease ovastacin. A proposed mechanism for the interaction of ovastacin and fetuin-B at the egg cell surface is based on an observation that in wild-type mouse oocytes, small amounts of ovastacin seeping from unfertilized eggs are inhibited by fetuin-B. Invading sperm will trigger the cortical degranulation reaction. Massive ovastacin release from cortical granules will override fetuin-B inhibition in the zona pellucida. Ovastacin will cleave ZP2 and the zona pellucida will harden. Thus, a mechanic protection of the fertilized egg and a block against polyspermy will be established. In contrast, in *fetuB*^{*-*/*-*} mice without fetuin-B, small amounts of ovastacin seeping from unfertilized eggs are not inhibited by fetuin-B. Thus, zona pellucida hardening (ZPH) will occur even in the absence of fertilization, and premature ZPH renders oocytes non-fertilizable (Stöcker et al., Biol. Chem. (2014), 395(10): 1195-1199; see Figure 1 therein).

Pre-mature release of ovastacin has also been suggested to be the reason for infertility of fetuin-B deficient female mice. Also, addition of fetuin-B (the naturally occurring ovastacin inhibitor) during IVF partially prevented ZPH and improved the fertilization rate (Körschgen et al., Molecular Human Reproduction (2017), 23(9): 607-616).

Therefore, the development of novel inhibitors of ovastacin can contribute to the treatment of mammal infertility and improving *in vitro* fertilization.

### Nematode Astacins

Nematode astacins are crucial for the development of nematodes (roundworms) and have specific roles in hatching, moulting and cuticle synthesis, as described by Stepek et al. (International Journal for Parasitology (2015), 45: 345-355). As noted therein, gastrointestinal (GI) nematodes cause chronic debilitating infections in livestock and humans worldwide, having a major economic impact on sheep farming resulting in a loss of appetite, weight loss, decreased wool, meat and milk production, as well as death. Current treatment is by use of anthelmintic drugs; however, multiple resistance to anthelmintics of the three major classes has now developed in veterinary parasites. Only a limited number of new drugs with novel modes of action have become available in recent years, thereby limiting prospects for effective control.

All nematodes are surrounded by an external protective structure called the cuticle. The cuticle functions as an exoskeleton and provides protection from the external environment during development, hence its importance for nematode survival. Synthesis of this structure is a complex, multi-step process, involving numerous enzymes. The cuticle is largely composed of collagens, which are homologous between the free-living nematode, C. *elegans,* and parasitic nematodes such as the major GI nematodes of sheep, *Teladorsagia circumcincta* and *Haemonchus contortus*. The process of cuticle biosynthesis has been studied in detail in *C*. *elegans,* with many of the crucial cuticle synthesizing enzymes and proteases also present in parasitic nematodes, suggesting that the cuticle biosynthesis process may be similar between *C*. *elegans* and its parasitic counterparts. Protease enzymes are essential for the continued development and survival of nematodes in the host and fall into the following main classes: aspartic, cysteine, metallo-, threonine and serine proteases. The astacin metalloprotease enzymes play an essential role in cuticle biosynthesis in *C*. *elegans.* These enzymes are zinc metallo-endopeptidases that are characterised by two conserved motifs in the N-terminal astacin domain: the zinc-binding active site and the methionine-turn (SxMHY). Binding of the zinc in the active site is essential for the catalytic activity of the enzyme; this zinc is pentacoordinated in a trigonal-bipyramidal geometry between the three histidine residues in the binding motif, the tyrosine in the methionine-turn and a water molecule. Functional roles for astacin proteases in parasitic nematodes include host tissue penetration by infective L3s (Williamson et al., Infect. Immun. (2006), 74: 961-967), cuticle formation and ecdysis (Gamble et al., J. Parasitol. (1989), 82: 197-202; Stepek et al., Int. J. Parasitol. (2010), 40: 533-542; Stepek at al., Parasitology (2011), 138: 237-248) and digestion (Gallego et al., Parasitol. Int. (2005), 54: 123-133).

There are 39 nematode astacin (NAS) metalloproteinases expressed in *C*. *elegans* (Stepek et al., International Journal for Parasitology (2015), 45: 345-355). All the *C*. *elegans* NAS have a similar domain arrangement. Among these, DPY-31 (also known as NAS-35) has similarities to the vertebrate procollagen C-proteinase bone morphogenetic protein 1 (BMP-1) mentioned above, which is critical for the assembly of collagen fibrils during cartilage and bone formation through its excision of the C-terminal domain of procollagen to form mature collagen. In *C*. *elegans,* DPY-31 is expressed throughout the life-cycle, particularly in the embryonic and larval stages, in most hypodermal cells, as well as rectal and vulvar epithelial cells (Novelli et al., Genetics (2004) 168, 1259-1273). The procollagens in DPY-31(e2770) mutants remain partially processed and cannot form mature collagens (Novelli et al., Genetics (2006), 172, 2253-2267.) Thus, DPY-31 plays a crucial role in cuticle formation and moulting process in *C*. *elegans.*

Identity of 74% has been noted between DPY-31 from *C. elegans* and *T. circumcincta,* as well as of 70% between *C. elegans* and *H. contortus* and of 89% between the proteins from *T. circumcincta* and *H. contortus.* The presence of DPY-31 orthologues throughout the entire nematode phylum supports a conserved, crucial role for this protease during nematode development. It has been further found that multiple compounds specifically developed to target procollagen C-proteinases (PCP) were important inhibitors also of recombinant nematodic DPY-31, thus being promising for development of new drugs to combat important nematode infections (Stepek et al., International Journal for Parasitology (2015), 45: 345-355). Finally, France et al. (Bioorganic & Medicinal Chemistry Letters (2015), 25: 5752-5755) reported several inhibitors of DPY-31 from the human filarial nematode *Brugia malayi* which are also active against DPY-31 from the parasitic gastrointestinal nematode of sheep *T. circumcincta.*

Thus, the zinc-dependent metzincin metalloproteases of the astacin family, more specifically procollagen C-proteinase (PCP) enzymes, meprins, ovastacin and/or nematode proteins, and even more specifically human or mammalian BMP-1, meprin α, meprin β and/or ovastacin as well as nematode DPY-31, can be considered highly relevant therapeutic targets, and there is a high demand for the development of new treatments of diseases and conditions associated therewith.

US 4,146,721 discloses pyrazol-4-acetic acid compounds, such as substituted pyrazol-4-acetic acid, its esters, amides, nitriles and their pharmaceutically acceptable salts and a method for the preparation of these compounds. These compounds are useful analgesics, anti-inflammatory, and antipyretics.

WO 2006114263 discloses imidazo [1, 2-a] pyridine derivatives useful as peptide deformylase (pdf) inhibitors.

E. Adiguzel et al. (Journal of Molecular Structure, vol. 1127, p. 403-12 (2017)) relates to the synthesis and characterization of two new hydroxamic acid derivatives and their metal complexes and the investigation on the keto/enol E/Z and hydroxamate / hydroximate forms.

### PROBLEMS TO BE SOLVED BY THE INVENTION

In view of the above, the present invention aims at the object of providing compounds and/or pharmaceutical compositions capable of inhibiting metalloproteinases of the astacin family; in particular procollagen C-proteinase (PCP) enzymes, meprins, ovastacin and/or nematode astacins; more particularly human or mammalian meprin α (such as human meprin α, hMeprin α), meprin β (such as human meprin β, hMeprin β), BMP-1 (such as human BMP-1, hBMP-1), ovastacin (such as human ovastacin, hOvastacin) and/or DPY-31 from nematodes (such as DPY-31 from *T. circumcincta* (tcDPY-31), *H. contortus* (hcDPY-31) and *B. malayi* (bmDPY-31).

Preferably, in order to mitigate potential side effects, the inhibitors and/or pharmaceutical compositions should be selective over further members of the metzincin superfamily including ADAMs (such as ADAM10 and ADAM17 (TACE)) and MMPs (such as MMP2, MMP9 and MMP13). Preferably, the inhibitors should selectively inhibit one or more of the enzymes selected from hMeprin α, hMeprin β, hBMP-1, hOvastacin, tcDPY-31, hcDPY-31 and bmDPY-31. Further preferably, the inhibitors should have acceptable drug-like properties.

A further object is to provide a pharmaceutical composition comprising an inhibitor according to any of the aforementioned objects that is suitable for administration to a subject in need thereof.

A further object is to provide a compound or a pharmaceutical composition for use in a method for treatment or prophylaxis of the human or animal body.

A further object is to provide a compound or a pharmaceutical composition for use in a method for treatment or prophylaxis of a subject suffering from or having risk of developing a disease or condition related to one or more of the above-mentioned metalloproteinases of the astacin family. Preferably, the disease or condition is associated with one or more of the enzymes selected from hMeprin α, hMeprin β, hBMP-1, hOvastacin, tcDPY-31, hcDPY-31 and bmDPY-31.

A further object is to provide a compound or a pharmaceutical composition for use in a method for treatment or prophylaxis of a subject suffering from or having risk of developing a disease or condition selected from Alzheimer's disease; nephritis; renal injury; renal ischemic injury; ischemic acute tubular necrosis; acute renal failure; bladder inflammation; inflammatory bowel disease (IBD); Crohn's disease; ulcerative colitis; chronic inflammation; colitis; fibrosis; fibrotic conditions; keloids; pulmonary hypertension; interstitial lung disease (ILD); cancer; and colorectal cancer.

A further object is to provide a compound or a pharmaceutical composition for use in a method for treatment or prophylaxis of a subject suffering from or having risk of developing a disease or condition selected from fibrosis; acute fibrotic disorders and conditions; chronical fibrotic disorders and conditions; fibrosis occurring in organs and/or accompanying diseases and conditions selected from hepatitis, liver cirrhosis, hypertension, myocardial infarction, heart failure, asthma, pulmonary hypertension, scleroderma, fibrotic skin and internal organs, diabetes, diabetes nephropathy, atherosclerosis and fibrotic blood vessels; hypertrophic dermal scarring; keloids; pulmonary fibrosis; acute CNS scarring following traumatic injury; neuronal regeneration following stroke or spinal cord injury; obliterative fibrosis of the hollow structures within grafts; chronic allograft rejection; wound healing disorders; post-surgical scarring; dermal scarring; fibrosis resulting from gynecological procedures; fibrosis after eye surgery; fibrosis following angioplasty; fibrosis following surgery on joints; preventing local invasion, recurrence and metastasis of malignant keratinocytes or squamous cell carcinomas (SCCs).

A further object is to provide a compound or a pharmaceutical composition for use in a method for treatment or prophylaxis of mammalian infertility and for therapeutic use for *in vitro* fertilization (IVF) treatment of a mammal.

A further object is to provide a compound or a pharmaceutical composition for use in a method for treatment or prophylaxis of a subject suffering from or having risk of developing a disease or condition selected from nematode infections; infections caused by *Teladorsagia circumcincta*; infections caused by *Haemonchus contortus*; and infections caused by *Brugia malayi.*

### SUMMARY OF THE INVENTION

As a solution to the above-formulated problems, the present invention provides a compound according to claim 1. In particular, the present invention provides a compound of the following Formula I, 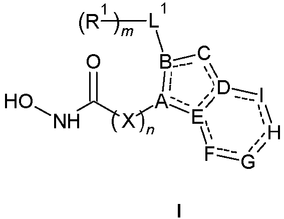 its individual enantiomers, its individual diastereoisomers, its hydrates, its solvates, its crystal forms, its individual tautomers or a pharmaceutically acceptable salt thereof, wherein:
A is independently selected from 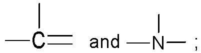
B is independently selected from 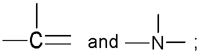
C is independently selected from -N= , 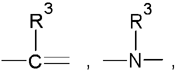 -O- and -S-;
F, if present, is independently selected from 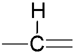 and -N= ;
G, if present, is independently selected from 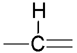 and -N= ;
H, if present, is independently selected from 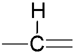 and -N= ;
I, if present, is independently selected from 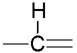 and -N= ;
wherein if F, G, H and I are present, then:
   D is 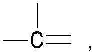
   E is independently selected from 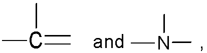 and
   the ring formed by D, E, F, G, H and I is substituted by *p* substituents represented by R²; otherwise if F, G, H and I are absent, then:
   D is independently selected from 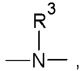 -N= and 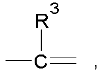 and
   E is independently selected from 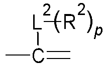 and -N= ;
each X is independently selected form C(R^{a})R^{b}, wherein one of the C(R^{a})R^{b} groups can be replaced by a NR^{a} group;
*n is* 1 or 2;
*m* is 0, 1, 2 or 3;
*p* is 1, 1, 2 or;
L¹ is phenyl;
L² is phenyl;
R¹ is independently selected from Cl, F, OH, OCH₃ and COOH, and/or two R¹ groups together form part of a 1,3-benzodioxol ring or a 2,3-dihydro-1,4-benzodioxin ring;
R² is COOH;
each R³ is independently selected from hydrogen, methyl, ethyl, propargyl, -CH₂COOH, benzyl, unsubstituted phenyl, and substituted phenyl selected from 3-carboxyphenyl and 4-carboxyphenyl; and
R^{a} and R^{b} are hydrogen;
with the provisos that
when the ring fragment 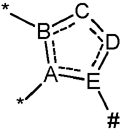 is represented by 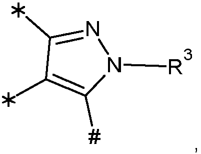 then:
   R³ is selected from hydrogen, methyl, benzyl, and substituted phenyl selected from 3-carboxyphenyl and 4-carboxyphenyl;
when the ring fragment larger than 0; and 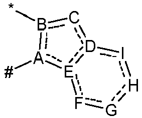 is represented by 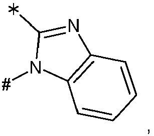 then at least one of *m* and *p* is
when the ring fragment 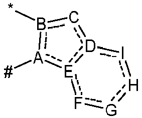 is represented by 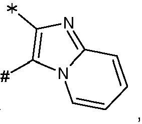 then m is larger than 0.

The present invention further provides a pharmaceutical composition comprising the compound as defined above and a pharmaceutically acceptable excipient.

The present invention further provides a compound or a pharmaceutical composition as defined above for use in a method for treatment or prophylaxis of the human or animal body by surgery or therapy, as well as methods for treatment or prophylaxis of the human or animal body by surgery or therapy comprising administering a therapeutically effective amount of the above compound or pharmaceutical composition to a subject in need thereof.

The present invention further provides a compound or a pharmaceutical as defined above for use in a method for therapy or prevention of diseases and conditions selected from:
(a) Alzheimer's disease; nephritis; renal injury; renal ischemic injury; ischemic acute tubular necrosis; acute renal failure; bladder inflammation; inflammatory bowel disease (IBD); Crohn's disease; ulcerative colitis; chronic inflammation; colitis; fibrosis; fibrotic conditions; keloids; pulmonary hypertension; interstitial lung disease (ILD); cancer; colorectal cancer;
(b) fibrosis; acute fibrotic disorders and conditions; chronical fibrotic disorders and conditions; fibrosis occurring in organs and/or accompanying diseases and conditions selected from hepatitis, liver cirrhosis, hypertension, myocardial infarction, heart failure, asthma, pulmonary hypertension, scleroderma, fibrotic skin and internal organs, diabetes, diabetes nephropathy, atherosclerosis and fibrotic blood vessels; hypertrophic dermal scarring; keloids; pulmonary fibrosis; acute CNS scarring following traumatic injury; neuronal regeneration following stroke or spinal cord injury; obliterative fibrosis of the hollow structures within grafts; chronic allograft rejection; wound healing disorders; post-surgical scarring; dermal scarring; fibrosis resulting from gynecological procedures; fibrosis after eye surgery; fibrosis following angioplasty; fibrosis following surgery on joints; preventing local invasion, recurrence and metastasis of malignant keratinocytes or squamous cell carcinomas (SCCs);
(c) mammalian infertility; therapeutic use for *in vitro* fertilization (IVF) treatment of a mammal;
(d) nematode infections; infections caused by *Teladorsagia circumcincta*; infections caused by *Haemonchus contortus*; and infections caused by *Brugia malayi,*
as well as, and/or methods for therapy or prevention of any of the preceding diseases or conditions, the method comprising administering a therapeutically effective amount of the above compound or pharmaceutical composition to a subject in need thereof.

### BRIEF DESCRIPTION OF THE DRAWINGS

**Fig. 1** shows structural alignment of Astacin-proteinases. hMeprin α (homology model, template pdb:4GWN), hMeprin β (X-ray, pdb: 4GWN), hOvastacin (homology model, template pdb: 3LQB), *Teladorsagia circumcincta* DPY-31 (tcDPY-31, homology model, template pdb: 6BTO), *Haemonchus contortus* DPY-31 (hcDPY-31, homology model, template pdb:6BTO), hBMP-1 (X-ray, pdb:6BTO).
**Fig. 2** shows an active site view of aligned proteinases (hMeprin α (homology model, template pdb:4GWN), hMeprin β (X-ray, pdb: 4GWN), hOvastacin (homology model, template pdb: 3LQB). Essential side chains for inhibitor binding are shown.
**Fig. 3** shows an active site view of aligned proteinases (hMeprin α (homology model, template pdb:4GWN), hMeprin β (X-ray, pdb: 4GWN)), *Teladorsagia circumcincta* DPY-31 (tcDPY-31, homology model, template pdb: 6BTO), *Haemonchus contortus* DPY-31 (hcDPY-31, homology model, template pdb:6BTO). Essential side chains for inhibitor binding are shown.
**Fig. 4** shows an active site view of aligned proteinases (hMeprin α (homology model, template pdb:4GWN), hMeprin β (X-ray, pdb: 4GWN), hBMP-1 (X-ray, pdb:6BTO). Essential side chain for inhibitor binding are shown.
**Fig. 5** shows a CLUSTAL O(1.2.4) multiple sequence alignment of hMeprin α, hMeprin β, hOvastacin, tcDPY-31 and hcDPY-31.

### DETAILED DESCRIPTION OF THE INVENTION

### Therapeutic Targets, Inhibitory Activity and Effects Achieved by the Invention

The present inventors have found that the compounds according to Formula I exhibit high inhibitory activity against various astacin metalloproteinases, in particular against hMeprin α and hMeprin β as evidenced by the experimental results shown herein (see Table 2). Additionally, as evidenced by the results shown in Table 3, the compounds also exhibit selectivity for astacin metalloproteinases while at the same time being significantly less active on further members of the metzincin superfamily including ADAMs (such as ADAM10 and ADAM17 (TACE)) and MMPs (such as MMP2, MMP9 and MMP13), thereby reducing the risk for potential side effects.

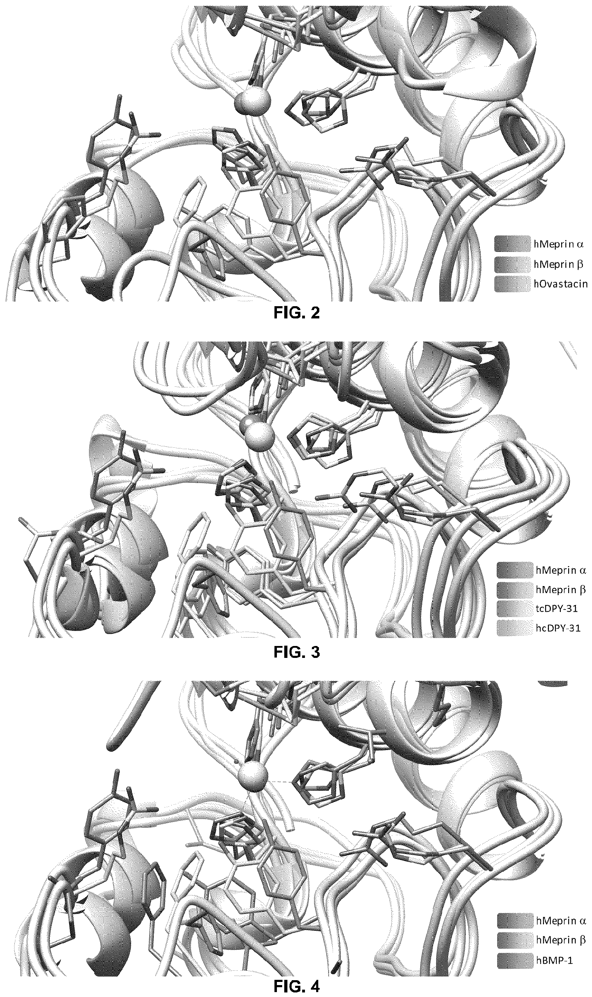

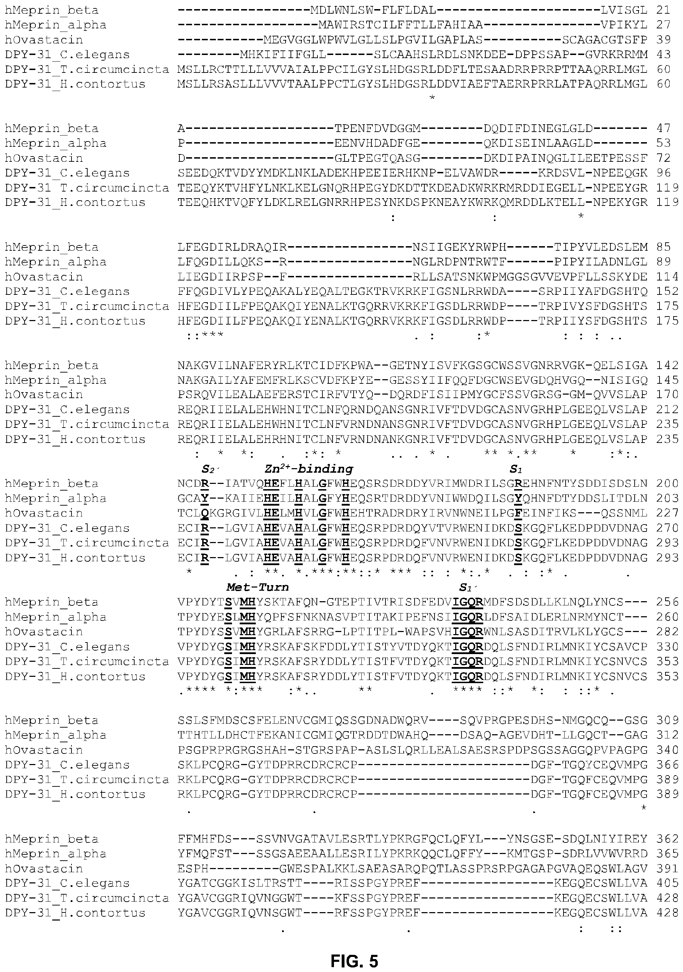

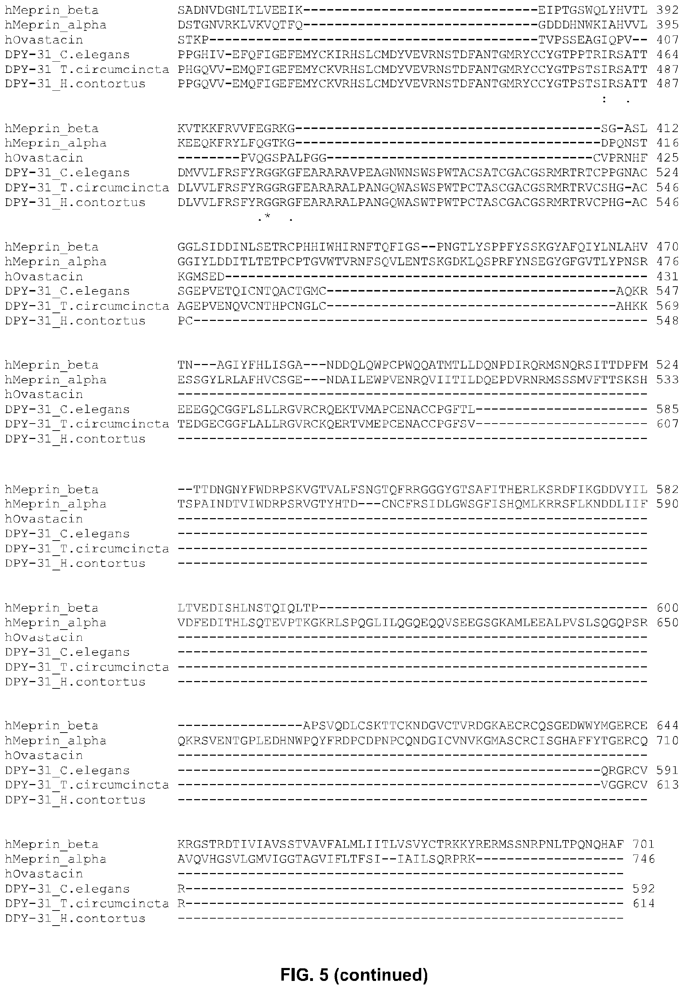

Additionally, further enzymes among the same family such as hOvastacin, tcDPY-31 and hcDPY-31 share an identical Zn²⁺ binding sequence in their respective active sites, an identical specificity pocket (S₁'), and a highly conserved methionine-containing turn (Met-turn, SxMHY) backing the zinc site, as can be seen from the sequence alignment shown in Fig. 5. Moreover, hMeprin α, hMeprin β, as hOvastacin, tcDPY-31 and hcDPY-31 strikingly similar overall three-dimensional structures (as shown in Fig. 1) and in particular of their active sites also responsible for inhibitor binding (as shown in Fig. 2-4).

Furthermore, earlier work has indicated that compounds that were known as highly effective inhibitors of procollagen C-proteinases (e.g., BMP-1) or meprins (actinonin) are also effective against nematode DPY-31 enzymes, such as tcDPY-31 and hcDPY-31 (Stepek et al., International Journal for Parasitology (2015), 45: 345-355) as well as tcDPY-31 and bmDPY-31 (France et al., Bioorganic & Medicinal Chemistry Letters (2015), 25: 5752-5755).

In view of the above evidence and the experimental results shown herein, all of the present compounds are useful as inhibitors of metalloproteinases of the astacin family; in particular procollagen C-proteinase (PCP) enzymes, meprins, ovastacin and/or nematode astacins; more particularly human or mammalian meprin α (such as human meprin α, hMeprin α), meprin β (such as human meprin β, hMeprin β), BMP-1 (such as human BMP-1, hBMP-1), ovastacin (such as human ovastacin, hOvastacin) and/or DPY-31 from nematodes (such as DPY-31 from *T. circumcincta* (tcDPY-31), *H. contortus* (hcDPY-31) and *B. malayi* (bmDPY-31).

The present invention provides a new class of inhibitors different in its physical chemical properties from compounds known from the prior art, such as secondary/tertiary amine or sulfonamide-based inhibitors. Also, the pharmacokinetic and pharmacodynamic properties of the new molecules are influenced thereby, which leads, e.g., to an improved activity against the target enzymes. Additionally, the present compounds are characterized by the presence of a flat aromatic or heteroaromatic moiety at the w-position of the respective alkylhydroxamic acids. The structural features and properties of the presently described compounds are clearly distinct from any previously described inhibitors of astacin metalloproteases or related enzymes.

### Compounds

Specifically, the present disclosure provides compounds to any one of the following aspects . <1> A compound according to the following Formula I, 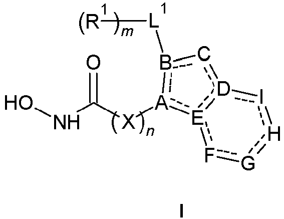 its individual enantiomers, its individual diastereoisomers, its hydrates, its solvates, its crystal forms, its individual tautomers or a pharmaceutically acceptable salt thereof, wherein:
A is independently selected from 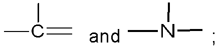 B is independently selected from 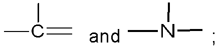
C is independently selected from -N= , 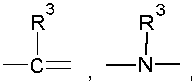 -O- and -S-;
F, if present, is independently selected from 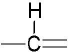 and -N= ;
G, if present, is independently selected from 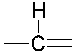 and -N= ;
H, if present, is independently selected from 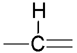 and -N= ;
I, if present, is independently selected from 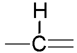 and -N= ;
wherein if F, G, H and I are present, then:
   D is 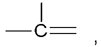
   E is independently selected from 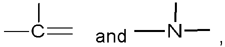 and
   the ring formed by D, E, F, G, H and I is substituted by *p* substituents represented by R²,;
otherwise if F, G, H and I are absent, then:
   D is independently selected from 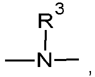 -N= and 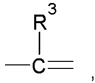 and
   E is independently selected from 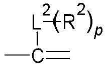 and -N= ;
each X is independently selected form C(R^{a})R^{b}, wherein one of the C(R^{a})R^{b} groups can be replaced by a NR^{a} group;
*n* is 1 or 2;
*m* is 0, 1, 2 or 3;
*p* is 0, 1, 2, or 3;
L¹ is phenyl;
L² is phenyl;
R¹ is independently selected from CI, F, OH, OCH₃ and COOH, and/or two R¹ groups together form part of a 1,3-benzodioxol ring or a 2,3-dihydro-1,4-benzodioxin ring;
R² is COOH;
each R³ is independently selected from hydrogen, methyl, ethyl, propargyl, -CH₂COOH, benzyl, unsubstituted phenyl, and substituted phenyl selected from 3-carboxyphenyl and 4-carboxyphenyl; and
R^{a} and R^{b} are hydrogen;
with the provisos that
   when the ring fragment 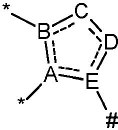 is represented by 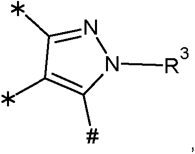 then:
   R³ is selected from hydrogen, methyl, benzyl, and substituted phenyl selected from 3-carboxyphenyl and 4-carboxyphenyl;
when the ring fragment 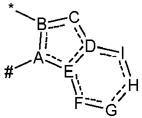 is represented by 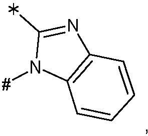 then at least one of *m* and *p* is larger than 0; and
when the ring fragment 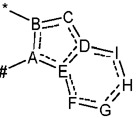 is represented by 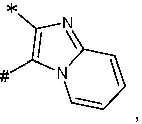 then m is larger than 0.

<2> The compound according to aspect 1, which is represented by the following Formula la: 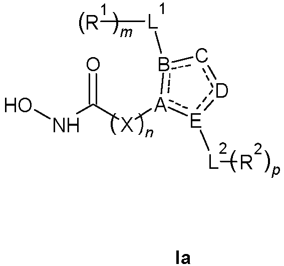
<3> The compound according to any one of aspects 1 or 2, wherein if F, G, H and I are absent, the ring fragment 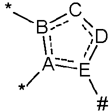 is represented by one of the following structures: 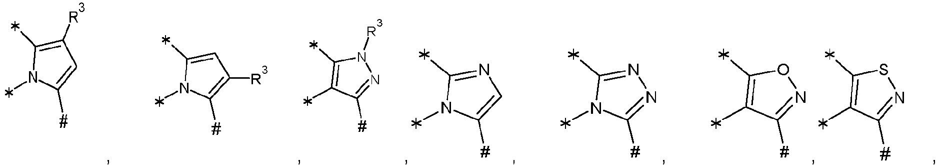 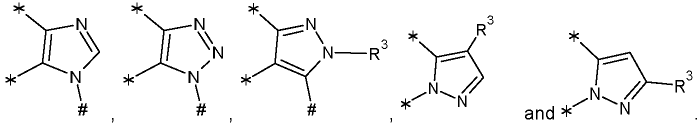
<4> The compound according to any one of aspects 1 to 3, which is represented by the following Formula Ib: 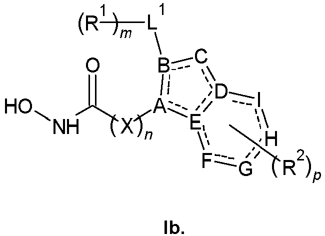
<5> The compound according to any one of aspects 1 to 4, wherein if F, G, H and I are present, the ring fragment is represented by one of the following structures: 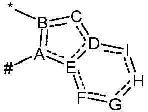 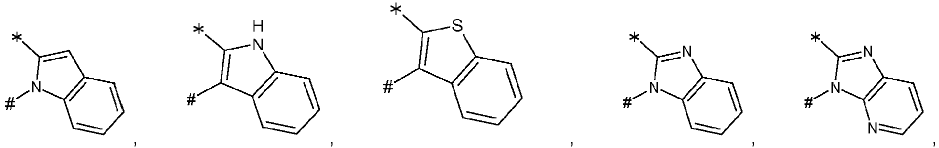 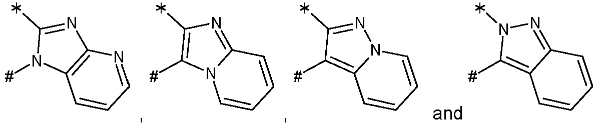
<6> The compound according to any one of aspects 1 to 5, wherein when the ring fragment 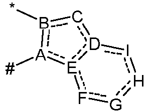 is represented by 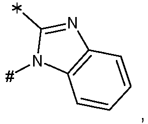 then at least one of *m* and *p* is larger than 0.
<7> The compound according to any of aspects 4 to 6, wherein *n =* 1.
<8> The compound according to any of aspects 1 to 7, wherein at least one or each of *m* and *p* is larger than 0.
<9> The compound according to any of aspects 1 to 8, wherein
   C is selected from -N= , 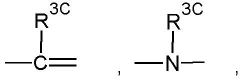 -O- and -S-;
   D is selected from 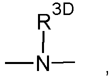 -N= and 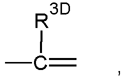 and
   R^{3C} and R^{3D} are the same or different from each other and each independently selected from the groups defined for R³ according to any one of the preceding aspects.
<10> The compound according to aspect 9, wherein R^{3C} and R^{3D} are different from each other.
<11> The compound according to any of aspects 1 to 10, wherein: each X is C(R^{a})R^{b}, wherein one of the C(R^{a})R^{b} groups can be replaced by a NR^{a} group; *n* is 1 or 2; *m* is 0, 1, 2 or 3; *p* is 0, 1, 2 or 3; L¹ is phenyl; L² is phenyl; R¹ is independently selected from CI, F, OH, CN, OCH₃ and COOH, and/or two R¹ groups together form part of a 1,3-benzodioxol ring or a 2,3-dihydro-1,4-benzodioxin ring; R² is independently selected from CI, F, OH, CN, OCH₃ and COOH, and/or two R² groups together form part of a 1,3-benzodioxol ring or a 2,3-dihydro-1,4-benzodioxin ring; R³ is selected from hydrogen, methyl, ethyl, propargyl, cyclopropyl, 2-aminoethyl, -CH₂COOH, -CH₂CN, benzyl, unsubstituted phenyl, and substituted phenyl selected from 3-carboxyphenyl and 4-carboxyphenyl; and R^{a} and R^{b} are hydrogen.
<12> The compound according to any of aspects 1 to 11, wherein X is C(R^{a})R^{b}; *n is* 1; and at least one of *m* and *p* is larger than 0.
<13> A compound having a structure selected from Table 2, its individual enantiomers, its individual diastereoisomers, its hydrates, its solvates, its crystal forms, its individual tautomers or a pharmaceutically acceptable salt thereof.
<14> A pharmaceutical composition comprising the compound according to any of aspects 1 to 13 and a pharmaceutically acceptable excipient.
<15> A compound according to any of aspects 1 to 13 or a pharmaceutical composition according to aspect 14 for use in a method for treatment of the human or animal body.
<16> A compound according to any of aspects 1 to 13 or a pharmaceutical composition according to aspect 14 for use in a method for therapy or prevention of diseases and conditions selected from Alzheimer's disease; nephritis; renal injury; renal ischemic injury; ischemic acute tubular necrosis; acute renal failure; bladder inflammation; inflammatory bowel disease (IBD); Crohn's disease; ulcerative colitis; chronic inflammation; colitis; fibrosis; fibrotic conditions; keloids; pulmonary hypertension; interstitial lung disease (ILD); cancer; and colorectal cancer.
<17> A compound according to any of aspects 1 to 13 or a pharmaceutical composition according to aspect 14 for use in a method for therapy or prevention of diseases and conditions selected from fibrosis; acute fibrotic disorders and conditions; chronical fibrotic disorders and conditions; fibrosis occurring in organs and/or accompanying diseases and conditions selected from hepatitis, liver cirrhosis, hypertension, myocardial infarction, heart failure, asthma, pulmonary hypertension, scleroderma, fibrotic skin and internal organs, diabetes, diabetes nephropathy, atherosclerosis and fibrotic blood vessels; hypertrophic dermal scarring; keloids; pulmonary fibrosis; acute CNS scarring following traumatic injury; neuronal regeneration following stroke or spinal cord injury; obliterative fibrosis of the hollow structures within grafts; chronic allograft rejection; wound healing disorders; post-surgical scarring; dermal scarring; fibrosis resulting from gynecological procedures; fibrosis after eye surgery; fibrosis following angioplasty; fibrosis following surgery on joints; preventing local invasion, recurrence and metastasis of malignant keratinocytes or squamous cell carcinomas (SCCs);
<18> A compound according to any of aspects 1 to 13 or a pharmaceutical composition according to aspect 14 for use in a method for therapy or prevention of diseases and conditions selected from mammalian infertility and therapeutic use *for in vitro* fertilization (IVF) treatment of a mammal.
<19> A compound according to any of aspects 1 to 13 or a pharmaceutical composition according to aspect 14 for use in a method for therapy or prevention of diseases and conditions selected from nematode infections; infections caused by *Teladorsagia circumcincta*; infections caused by *Haemonchus contortus*; and infections caused by *Brugia malayi.*

### Definitions

As used herein, the symbol 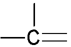 represents an sp² carbon atom capable of being connected with three further atoms, and should be understood to denote any orientation within the respective molecular structure, e.g., also 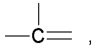 with the proviso that the double bond must be part of the respective planar ring system (ABCDE or ABCDEFGHI). This applies analogously to the symbols 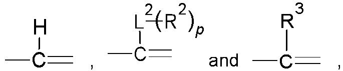 the only difference in these cases being that the respective sp² carbon atom is capable of being connected with two further atoms of the respective planar ring system, whereas the group H, L²(R²)*ₚ* and R³, respectively, are substituents of the respective planar ring system (ABCDE or ABCDEFGHI). As used herein, the symbol -N= represents an sp² nitrogen atom capable of being connected with two further atoms, and should be understood to denote any orientation within the respective molecular structure, e.g., also =N- , with the proviso that the double bond must be part of the respective planar ring system (ABCDE or ABCDEFGHI). As used herein, the symbol 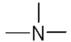 represents an sp³ nitrogen atom capable of being connected with three further atoms, and should be understood to comprise any orientation within the respective molecular structure. This applies analogously to the symbol 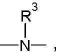 the only difference in this cases being that the sp³ nitrogen atom is capable of being connected with two further atoms of the respective planar ring system, whereas the group R³ is a substituent of the respective planar ring system.

The expression "alkyl" as used herein, unless specifically limited, denotes a C₁₋₁₂ alkyl group, suitably a C₁₋₈ alkyl group, e.g. C₁₋₆ alkyl group, e.g. C₁₋₄ alkyl group. Alkyl groups may be straight chain or branched. Suitable alkyl groups include, for example, methyl, ethyl, propyl (e.g. n-propyl and isopropyl), butyl (e.g. n-butyl, iso-butyl, sec-butyl and tert-butyl), pentyl (e.g. n-pentyl), hexyl (e.g. n-hexyl), heptyl (e.g. n-heptyl) and octyl (e.g. n-octyl). The term "alkyl" also comprises cycloalkyl groups. The expression "cycloalkyl", unless specifically limited, denotes a C₃₋₁₀ cycloalkyl group (i.e. 3 to 10 ring carbon atoms), more suitably a C₃₋₈ cycloalkyl group, e.g. a C₃₋₆ cycloalkyl group. Exemplary cycloalkyl groups include cyclopropyl, cyclobutyl, cyclopentyl, cyclohexyl, cycloheptyl and cyclooctyl. A most suitable number of ring carbon atoms is three to six.

The expression "heteroalkyl", unless specifically limited, refers to an alkyl group wherein one or more carbon atoms, preferably 1, 2 or 3, are replaced by heteroatoms selected from N, S and O.

The expressions "carbocyclyl" and "carbocyclic", unless specifically limited, denote any ring system in which all the ring atoms are carbon, and which contains between three and twelve ring carbon atoms, suitably between three and ten carbon atoms and more suitably between three and eight carbon atoms. Carbocyclyl groups may be saturated or partially unsaturated, but do not include aromatic rings. Examples of carbocyclyl groups include monocyclic, bicyclic, and tricyclic ring systems, in particular monocyclic and bicyclic ring systems. Other carbocyclic groups include bridged ring systems (e.g. bicyclo[2.2.1]heptenyl). A specific example of a carbocyclyl group is a cycloalkyl group. A further example of a carbocyclyl group is a cycloalkenyl group.

The expression "aryl", unless specifically limited, denotes a C₆₋₁₂ aryl group, suitably a C₆₋₁₀ aryl group, more suitably a C₆₋₈ aryl group. Aryl groups will contain at least one aromatic ring (e.g. one, two or three rings). An example of a typical aryl group with one aromatic ring is phenyl. An example of a typical aryl group with two aromatic rings is naphthyl.

The expressions "heterocyclyl" and "heterocyclyc", unless specifically limited, refer to a carbocyclyl group wherein one or more (e.g. 1, 2 or 3) ring atoms are replaced by heteroatoms selected from N, S and O. A specific example of a heterocyclyl group is a cycloalkyl group (e.g. cyclopentyl or more particularly cyclohexyl) wherein one or more (e.g. 1, 2 or 3, particularly 1 or 2, especially 1) ring atoms are replaced by heteroatoms selected from N, S or O. Exemplary heterocyclyl groups containing one hetero atom include pyrrolidine, tetrahydrofuran and piperidine, and exemplary heterocyclyl groups containing two hetero atoms include morpholine and piperazine. A further specific example of a heterocyclyl group is a cycloalkenyl group (e.g. a cyclohexenyl group) wherein one or more (e.g. 1, 2 or 3, particularly 1 or 2, especially 1) ring atoms are replaced by heteroatoms selected from N, S and O. An example of such a group is dihydropyranyl (e.g. 3,4-dihydro-2H-pyran-2-yl-).

The expression "heteroaryl", unless specifically limited, denotes an aryl residue, wherein one or more (e.g. 1, 2, 3, or 4, suitably 1 , 2 or 3) ring atoms are replaced by heteroatoms selected from N, S and O, or else a 5-membered aromatic ring containing one or more (e.g. 1 , 2, 3, or 4, suitably 1 , 2 or 3) ring atoms selected from N, S and O. Heteroaryl groups represent a particular subtype within the general class of "heterocyclyl" or "heterocyclyc" groups. Exemplary monocyclic heteroaryl groups having one heteroatom include: five membered rings (e.g. pyrrole, furan, thiophene); and six membered rings (e.g. pyridine, such as pyridin-2-yl, pyridin-3-yl and pyridin-4-yl). Exemplary monocyclic heteroaryl groups having two heteroatoms include: five membered rings (e.g. pyrazole, oxazole, isoxazole, thiazole, isothiazole, imidazole, such as imidazol-1-yl, imidazol-2-yl imidazol-4-yl); six membered rings (e.g. pyridazine, pyrimidine, pyrazine). Exemplary monocyclic heteroaryl groups having three heteroatoms include: 1,2,3-triazole and 1,2,4-triazole. Exemplary monocyclic heteroaryl groups having four heteroatoms include tetrazole. Exemplary bicyclic heteroaryl groups include: indole (e.g. indol-6-yl), benzofuran, benzthiophene, quinoline, isoquinoline, indazole, benzimidazole, benzothiazole, quinazoline and purine.

The expressions "alkoxyaryl", "carboxyaryl", "cyanoaryl", "haloaryl", "hydroxyaryl" and "heteroarylaryl", unless specifically limited, denote an aryl residue which is substituted by at least one alkoxy, carboxy, cyano, halo, hydroxy and heteroaryl group, respectively.

The expressions "alkoxyheteroaryl", "carboxyheteroaryl", "cyanoheteroaryl", "haloheteroaryl" and "hydroxyheteroaryl", unless specifically limited, denote a heteroaryl residue which is substituted by at least one alkoxy, carboxy, cyano, halo, and hydroxy group, respectively.

The expression "alk", for example in the expressions "alkoxy", "haloalkyl" should be interpreted in accordance with the definition of "alkyl". Exemplary alkoxy groups include methoxy, ethoxy, propoxy (e.g. n-propoxy), butoxy (e.g. n-butoxy), pentoxy (e.g. n-pentoxy), hexoxy (e.g. n-hexoxy), heptoxy (e.g. n-heptoxy) and octoxy (e.g. n-octoxy). Exemplary haloalkyl groups include fluoroalkyl e.g. CF₃; exemplary haloalkoxy groups include fluoroalkyl e.g. OCF₃.

The term "halogen" or "halo" comprises fluorine (F), chlorine (CI), bromine (Br) and iodine (I).

The terms "hydrogen" or "H" as used herein encompass all isotopes of hydrogen, in particular protium (¹H) and deuterium (²H, also denoted as D).

The term "optionally substituted" refers to optional substitution by one or more groups independently selected from amino, halogen, cyano, hydroxy, carboxy, -C(O)O(alkyl), -C(O)NH₂, -C(O)NH(alkyl), alkylsulfono, a functional group having an acidic hydrogen, alkoxy, alkyl, alkenyl, alkynyl, cycloalkyl, cycloalkenyl, aryl, arylalkyl, heterocyclyl, heteroaryl and heteroarylalkyl, each of which can be further substituted by one or more groups independently selected from halogen, carboxy, cyano, alkyl, alkoxy and hydroxy; wherein *preferably*, at each occurrence, said aryl is a C₆ aryl group; said heterocyclyl is a heterocyclic group comprising 1 to 4 ring heteroatoms selected from N, S and O; said heteroaryl is a C₃₋₅ aromatic heterocyclic group comprising 1 to 3 ring heteroatoms selected from N, S and O; said alkyl or alk is a C₁₋₂ alkyl group; said alkenyl is a C₂ group comprising at least one C=C bond; said alkynyl is a C₂₋₃ group comprising at least one C=C bond; said cycloalkyl is a C₃₋₆, monocyclic or bicyclic alkyl group; said cycloalkenyl is a C₅₋₆ carbocyclic group comprising at least one C=C bond.

As used herein, the meaning of the term "comprising" encompasses three alternatives, namely "comprising", "consisting of" and "consisting essentially of".

### Stereoisomers

All possible stereoisomers of the claimed compounds are included in the present invention. Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Where the processes for the preparation of the compounds according to the invention give rise to a mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their components enantiomers by standard techniques, such as the formation of diastereomeric pairs by salt formation with an optically active acid, such as (-)-di-p-toluoyl-d-tartaric acid and/or (+)-di-p-toluoyl-l-tartaric acid followed by fractional crystallization and regeneration of the free base, or by salt formation with an optically active base, such as quinine, quinidine, quinotoxine, cinkotoxine, (*S*)-phenylethylamine, (1*R*,2*S*)-ephedrine, (*R*)-phenylglycinol, (*S*)-2-aminobutanol, followed by fractional crystallization and regeneration of the free acid. The compounds may also be resolved by formation of diastereomeric esters or amides, followed by chromatographic separation and removal of the chiral auxiliary. Alternatively, the compounds may be resolved using a chiral HPLC column.

### Polymorph Crystal Forms, Solvates, Hydrates

Furthermore, some of the individual crystalline forms of the compounds may exist as polymorphs and as such are intended to be included in the present invention. In addition, some of the compounds may form solvates with water (i.e. hydrates) or common organic solvents, and such solvates are also intended to be encompassed within the scope of this invention. The compounds, including their salts, can also be obtained in the form of their hydrates, or include other solvents used for their crystallization. In view of the close relationship between the free compounds and the compounds in the form of their salts, hydrates or solvates, whenever a compound is referred to in this context, a corresponding salt, solvate or polymorph is also intended, provided such is possible or appropriate under the circumstances.

### Tautomers

As used herein, the term "tautomer" refers to the migration of protons between adjacent single and double bonds. The tautomerization process is reversible. Compounds described herein can undergo any possible tautomerization that is within the physical characteristics of the compound.

### Pharmaceutically Acceptable Salts

As used herein, the term "pharmaceutically acceptable" embraces both human and veterinary use. For example, the term "pharmaceutically acceptable" embraces a veterinarily acceptable compound or a compound acceptable in human medicine and health care.

Salts, hydrates and solvates of the compounds of Formula I and physiologically functional derivatives thereof which are suitable for use in medicine are those wherein the counter-ion or associated solvent is pharmaceutically acceptable. However, salts, hydrates and solvates having non-pharmaceutically acceptable counter-ions or associated solvents are within the scope of the present invention, for example, for use as intermediates in the preparation of other compounds and their pharmaceutically acceptable salts, hydrates and solvates.

Suitable salts according to the invention include those formed with either organic and inorganic acids or bases. Pharmaceutically acceptable acid addition salts include those formed from hydrochloric, hydrobromic, sulfuric, nitric, citric, tartaric, phosphoric, lactic, pyruvic, acetic, trifluoroacetic, triphenylacetic, sulfamic, sulfanilic, succinic, oxalic, fumaric, maleic, malic, mandelic, glutamic, aspartic, oxaloacetic, methanesulfonic, ethanesulfonic, arylsulfonic (for example p-toluenesulfonic, benzenesulfonic, naphthalenesulfonic or naphthalenedisulfonic), salicylic, glutaric, gluconic, tricarballylic, cinnamic, substituted cinnamic (for example, phenyl, methyl, methoxy or halo substituted cinnamic, including 4-methyl and 4-methoxycinnamic acid), ascorbic, oleic, naphthoic, hydroxynaphthoic (for example 1- or 3-hydroxy-2-naphthoic), naphthaleneacrylic (for example naphthalenes-acrylic), benzoic, 4-methoxybenzoic, 2-or 4-hydroxybenzoic, 4-chlorobenzoic, 4-phenylbenzoic, benzeneacrylic (for example 1,4-benzenediacrylic), isethionic acids, perchloric, propionic, glycolic, hydroxyethanesulfonic, pamoic, cyclohexanesulfamic, salicylic, saccharinic and trifluoroacetic acid. Pharmaceutically acceptable base salts include ammonium salts, alkali metal salts such as those of sodium and potassium, alkaline earth metal salts such as those of calcium and magnesium and salts with organic bases such as dicyclohexylamine and *N*-methyl-*D*-glucamine.

All pharmaceutically acceptable acid addition salt forms of the compounds of the present invention are intended to be embraced by the scope of the present invention.

### Pharmaceutical Compositions

The pharmaceutical composition according to the present invention comprises a compound as described above and a pharmaceutically acceptable excipient.

As used herein, the term "pharmaceutical composition" is intended to encompass a product comprising the claimed compounds in the therapeutically effective amounts, as well as any product that results, directly or indirectly, from combinations of the claimed compounds. As used herein, the term "excipient" refers to a carrier, a binder, a disintegrator and/or a further suitable additive for galenic formulations, for instance, for liquid oral preparations, such as suspensions, elixirs and solutions; and/or for solid oral preparations, such as, for example, powders, capsules, gelcaps and tablets. Carriers, which can be added to the mixture, include necessary and inert pharmaceutical excipients, including, but not limited to, suitable suspending agents, lubricants, flavorants, sweeteners, preservatives, coatings, granulating agents, dyes, and coloring agents.

### Therapeutic Applications

The present disclosure provides a compound, e.g., a compound of any one of the above aspects <1>-<24>, and/or a pharmaceutical composition as described above for use in a method for treatment of the human or animal body. The present disclosure also provides a method for treatment of the human or animal body wherein the method comprises administration of a therapeutically effective amount of said compound or composition to a subject in need thereof.

The present disclosure further provides a compound and/or a pharmaceutical composition as described herein, e.g., a compound of any one of the above aspects <1>-<24>, for use in a method for therapy or prophylaxis of diseases and conditions associated with Meprin α and/or Meprin β; as well as a method for therapy or prophylaxis of such diseases and conditions wherein the method comprises administration of a therapeutically effective amount of said compound or composition to a subject in need thereof. As explained in the background section above, such diseases and conditions associated with Meprin α and/or Meprin β include Alzheimer's disease; nephritis; renal injury; renal ischemic injury; ischemic acute tubular necrosis; acute renal failure; bladder inflammation; inflammatory bowel disease (IBD); Crohn's disease; ulcerative colitis; chronic inflammation; colitis; fibrosis; fibrotic conditions; keloids; pulmonary hypertension; interstitial lung disease (ILD); cancer; and colorectal cancer.

The present disclosure further provides a compound and/or a pharmaceutical composition as described herein, e.g., a compound of any one of the above aspects <1>-<24>, for use in a method for therapy or prophylaxis of diseases and conditions associated with BMP-1; as well as a method for therapy or prophylaxis of such diseases and conditions wherein the method comprises administration of a therapeutically effective amount of said compound or composition to a subject in need thereof. As explained in the background section above, such diseases and conditions associated with associated with BMP-1 include fibrosis; acute fibrotic disorders and conditions; chronical fibrotic disorders and conditions; fibrosis occurring in organs and/or accompanying diseases and conditions selected from hepatitis, liver cirrhosis, hypertension, myocardial infarction, heart failure, asthma, pulmonary hypertension, scleroderma, fibrotic skin and internal organs, diabetes, diabetes nephropathy, atherosclerosis and fibrotic blood vessels; hypertrophic dermal scarring; keloids; pulmonary fibrosis; acute CNS scarring following traumatic injury; neuronal regeneration following stroke or spinal cord injury; obliterative fibrosis of the hollow structures within grafts; chronic allograft rejection; wound healing disorders; post-surgical scarring; dermal scarring; fibrosis resulting from gynaecological procedures; fibrosis after eye surgery; fibrosis following angioplasty; fibrosis following surgery on joints; and preventing local invasion, recurrence and metastasis of malignant keratinocytes or squamous cell carcinomas (SCCs).

The present disclosure further provides a compound and/or a pharmaceutical composition as described herein, e.g., a compound of any one of the above aspects <1>-<24>, for use in a method for therapy or prophylaxis of diseases and conditions associated with ovastacin; as well as a method for therapy or prophylaxis of such diseases and conditions wherein the method comprises administration of a therapeutically effective amount of said compound or composition to a subject in need thereof. As explained in the background section above, such diseases and conditions associated with ovastacin include mammalian infertility; and therapeutic use for *in vitro* fertilization (IVF) treatment of a mammal. Typical candidates for such treatment can be subjects (mammals) suffering from or suspected of suffering from infertility. Preferably, the subject is a female. Further preferably, the subject is a female human. According to the WHO-ICMART revised glossary, infertility in the case of humans can be defined as "a disease of the reproductive system defined by the failure to achieve a clinical pregnancy after 12 months or more of regular unprotected sexual intercourse." Additionally, the present compounds can be administered to females undergoing assisted reproductive treatments such as *in vitro* fertilization, e.g., for stimulating fertilization. Additionally, the present compounds can be used as a method for improving the fertilization rate for a subject undergoing assisted reproduction treatment or procedure, e.g., by administering a therapeutically effective amount of said compound or composition to a subject undergoing such treatment, and/or by contacting an oocyte *in vitro* with a composition comprising a compound as described herein.

The term "*in vitro* fertilization (IVF)" may refer to a method comprising collecting an ovum, fertilizing the ovum *in vitro* with a spermatozoon and, when cleavage has progressed to a certain degree, inserting the ovum into the uterine cavity, i.e. it may include the processes of ovulation induction, ovum collection, *in vitro* fertilization and culture, and embryo transfer.

The term "subject" as used herein refers to an animal, preferably a mammal, most preferably a human.

The term "therapeutically effective amount" as used herein means that amount of active compound or pharmaceutical agent that elicits the biological or medicinal response in a tissue system, animal or human being sought by a researcher, veterinarian, medical doctor or other clinician, which includes alleviation of the symptoms of the disease or disorder being treated.

### EXAMPLES

### I. Description of Synthetic Methods

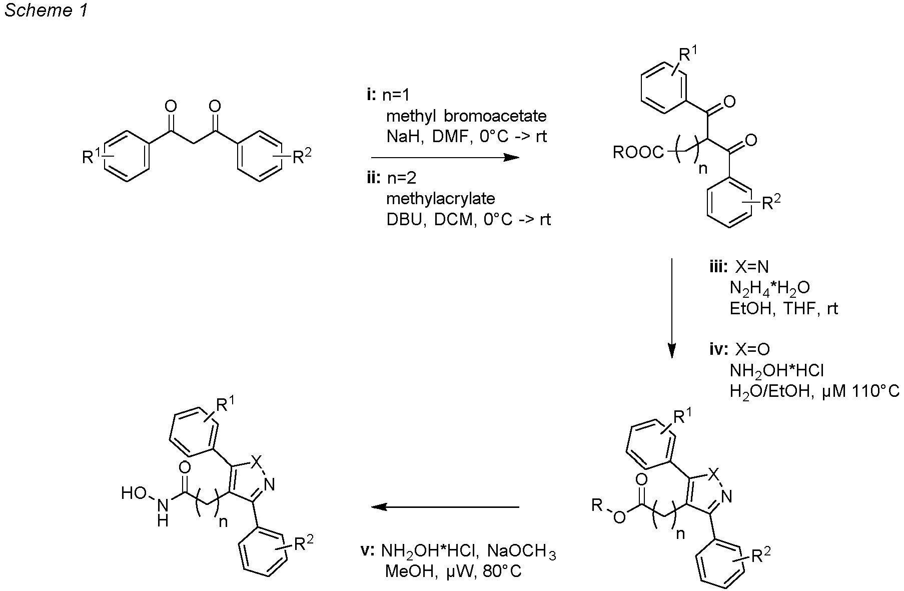

### Method i:

The respective 1,3-diaryl-1,3-propanedione or azole derivative (1 eq) was dissolved in DMF (c=0.5 M), cooled to 0°C and treated with sodium hydride (1.2 eq). After 30 min, methyl bromoacetate or another suitable alkyl halide (1.1 eq) was added dropwise. The mixture was allowed to warm up to room temperature and stirred for 12 hours. The reaction was stopped by addition of water and extracted with EtOAc (3x30 ml). The combined organic layers were dried over Na₂SO₄ and evaporated. The residue was purified by flash chromatography (silica, heptane/EtOAc gradient).

### Method ii:

The respective 1,3-diaryl-1,3-propanedione (1 eq) was dissolved in dichloromethane (c=0.2 M), cooled to 0°C and treated with 1,8-diazabicyclo[5.4.0]undec-7-en (DBU, 0.2 eq). After 15 min, methyl acrylate (2 eq.) dissolved in DCM was added dropwise over 15 min. After complete addition, the reaction was warmed to room temperature and stirred for 48 hours. The reaction was stopped by addition of saturated aqueous NaHCO₃. The organic layer was separated, and the aqueous phase was extracted with EtOAc (3x25 ml). The combined organic layers were dried over Na₂SO₄ and evaporated. The residue was purified by flash chromatography (silica, heptane/EtOAc gradient).

### Method iii:

A compound obtained either by method A or B (1 eq) was dissolved in an EtOH/THF mixture (2:1, v/v, c=0.07 M). Hydrazine monohydrate (5 eq) was added and the mixture was stirred at room temperature. Upon complete consumption of the starting material (~4-5 hours), the volatiles were evaporated. The remains were taken up with water, acidified by means of diluted aqueous HCl and extracted with EtOAc (3x25 ml). The combined organic layers were dried over Na₂SO₄ and evaporated. The residue was purified by flash chromatography (silica, heptane/EtOAc gradient).

### Method iv:

A compound obtained either by method A or B (1 eq) was dissolved in an EtOH/water mixture (3:2, v/v, c=0.4 M). Hydroxylamine hydrochloride (1 eq) was added and the mixture was heated in a microwave at 110°C for 20 minutes. After cooling, the mixture was poured into iced water and extracted with DCM (2x25 ml). The combined organic layers were dried over Na₂SO₄ and evaporated. The residue was purified by flash chromatography (silica, heptane/EtOAc gradient).

### Method v:

The respective ester derivative (1 eq) was dissolved in MeOH (5 ml), treated with NaOCH₃ (6 eq) and hydroxylamine hydrochloride (3 eq). The mixture was heated in a microwave at 80°C for 10 minutes. The volatiles were evaporated. The remains were taken up in water, acidified by means of diluted aqueous HCl and extracted with EtOAc (3x25 ml). The combined organic layers were dried over Na₂SO₄ and evaporated. The residue was purified by semi-preparative HPLC.

### Example 1: 2-(3,5-Diphenyl-1H-pyrazol-4-yl)ethanehydroxamic acid

The compound was synthesized according to methods i, iii and v as described above. Yield (last step): 83 mg (40%); ESI-MS: m/z 294.5 [M+H]⁺; HPLC (gradient 1): rt 12.03 min (>99%); ¹H NMR, 400 MHz, DMSO d₆: δ 3.32 (s, 1.8H), 3.63 (s, 0.2H), 7.38-7.42 (m, 2H), 7.46-7.49 (m, 4H), 7.64-7.66 (m, 4H), 10.09 (br s, 0.1H), 10.62 (br s, 0.9H) mixture of cis-trans isomers.

### Example 2: 2-(3,5-Diphenylisoxazol-4-yl)ethanehydroxamic acid

The compound was synthesized according to methods i, iv and v as described above. Yield (last step): 32 mg (35%); ESI-MS: m/z 295.1 [M+H]⁺, 317.2 [M+Na]⁺; HPLC (gradient 1): rt 14.56 min (>99%); ¹H NMR, 400 MHz, DMSO d₆: δ 3.41 (s, 2H), 7.55-7.62 (m, 6H), 7.68-7.70 (m, 2H), 7.79-7.82 (m, 2H), 10.77 (s, 1H).

### Example 3: 3-(3,5-Diphenyl-1H-pyrazol-4-yl)propanehydroxamic acid

The compound was synthesized according to methods ii, iii and v as described above. Yield (last step): 58 mg (19%); ESI-MS: m/z 308.4 [M+H]⁺; HPLC (gradient 1): rt 15.52 min (96.8%); ¹H NMR, 400 MHz, DMSO d₆: δ 2.06-2.11 (m, 2H), 2.93-2.97 (m, 2H), 7.41 (t, 2H, ³*J* = 7.3 Hz), 7.50 (t, 4H, ³*J* = 7.6 Hz), 7.64 (d, 4H, ³*J* = 7.8 Hz), 10.33 (br s, 1H).

### Example 4: 3-(3,5-Diphenylisoxazol-4-yl)propanehydroxamic acid

The compound was synthesized according to methods ii, iv and v as described above. Yield (last step): 11 mg (22%); ESI-MS: m/z 309.4 [M+H]⁺, 331.4 [M+Na]⁺; HPLC (gradient 1): rt 14.93 min (>99%); ¹H NMR, 400 MHz, DMSO d₆: δ 2.09-2.13 (m, 2H), 2.95-2.99 (m, 2H), 7.57-7.63 (m, 2H), 7.69-7.71 (m, 2H), 7.81-7.84 (m, 2H), 10.36 (br s, 1H).

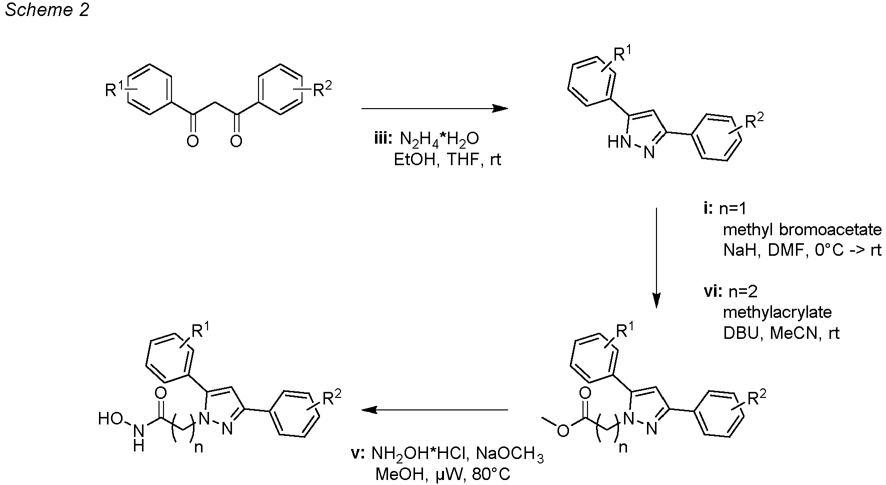

### Method vi:

The respective diarylpyrazole (1 eq) obtained by method C as described above, or another suitable azole derivative, was dissolved in acetonitrile (c=1.7 M) and treated with DBU (0.5 eq) and methyl acrylate (1.5 eq). The mixture was stirred at room temperature overnight. The volatiles were evaporated, and the remains were dissolved in EtOAc. Water was added, and the aqueous layer was extracted with EtOAc (3x25 ml). The combined organic layers were dried over Na₂SO₄ and evaporated. The residue could be used without further purification.

### Example 5: 2-(3,5-Diphenylpyrazol-1-yl)ethanehydroxamic acid

The compound was synthesized according to methods iii, i and v as described above. Yield (last step): 141 mg (57%); ESI-MS: m/z 294.4 [M+H]⁺; HPLC (gradient 1): rt 15.63 min (97.5%); ¹H NMR, 400 MHz, DMSO d₆: δ 4.68 (s, 1.8H), 5.04 (br s, 0.2H), 6.91 (s, 1H), 7.31-7.35 (m, 1H), 7.41-7.55 (m, 5H), 7.65-7.68 (m, 2H), 7.83-7.85 (m, 2H), 9.12 (br s, 0.9H), 9.36 (br s, 0.1H), 10.35 (br s, 0.1H), 10.93 (br s, 0.9H) mixture of cis-trans isomers.

### Example 6: 3-(3,5-Diphenylpyrazol-1-yl)propanehydroxamic acid

The compound was synthesized according to methods iii, vi and v as described above. Yield (last step): 171 mg (66%); ESI-MS: m/z 308.4 [M+H]⁺; HPLC (gradient 1): rt 17.68 min (>99%); ¹H NMR, 400 MHz, DMSO d₆: δ 2.66 (t, 2H, ³*J* = 7.2 Hz), 4.32 (t, 2H, ³*J* = 7.2 Hz), 6.84 (s, 1H), 7.30-7.34 (m, 1H), 7.41-7.45 (m, 2H), 7.47-7.57 (m, 3H), 7.60-7.63 (m, 2H), 7.84-7.86 (m, 2H), 9.97 (br s, 0.1H), 10.53 (br s, 0.9H) mixture of cis-trans isomers.

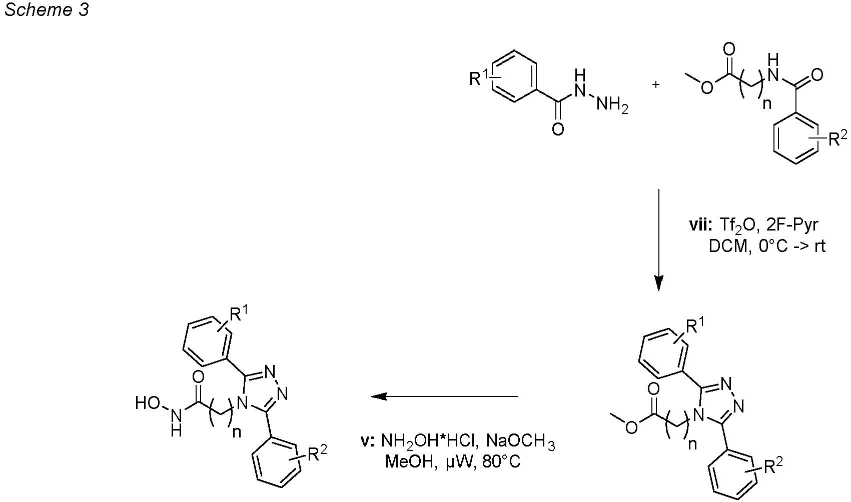

### Method vii:

The respective benzamide derivative (1 eq) and 2-fluoro-pyridine (1.1 eq) were dissolved in DCM (3 ml) in a sealed tube under argon atmosphere. The mixture was chilled on an ice bath and trifluoromethanesulfonic anhydride (1.1 eq) was added slowly via syringe. After further stirring at 0°C for 45 minutes, the respective benzhydrazide was added. After 45 minutes at room temperature, the mixture was heated in a microwave at 140°C for 2 hours. After cooling, saturated aqueous NaHCO₃ was added and extracted with DCM (3x25 ml). The combined organic layers were dried over Na₂SO₄ and evaporated. The residue was purified by flash chromatography (silica, chloroform/methanol gradient).

### Example 7: 2-(3,5-Diphenyl-1,2,4-triazol-4-yl)ethanehy-droxamic acid

The compound was synthesized according to methods vii and v as described above. Yield (last step): 45 mg (53%); ESI-MS: m/z 295.3 [M+H]⁺; HPLC (gradient 1): rt 7.73 min (98.2%); ¹H NMR, 400 MHz, DMSO d₆: δ 4.56 (s, 1.5 H), 4.85 (s, 0.5 H), 7.58-7.70 (m, 10 H), 10.50 (br s, 0.3 H), 10.81 (br s, 0.7 H) mixture of cis-trans isomers.

### Example 8: 3-(3,5-Diphenyl-1,2,4-triazol-4-yl)propane-hydroxamic acid

The compound was synthesized according to methods vii and v as described above. Yield (last step): 39 mg (37%); ESI-MS: m/z 309.4 [M+H]⁺; HPLC (gradient 1): rt 8.59 min (97.0%); ¹H NMR, 400 MHz, DMSO d₆: δ 2.10 (t, 2H, ³*J* = 7.6 Hz), 4.39 (t, 2H, ³*J* = 7.5 Hz), 7.61-7.63 (m, 6H), 7.75-7.78 (m, 4H), 9.89 (br s, 0.1H), 10.28 (br s, 0.9H) mixture of cis-trans isomers.

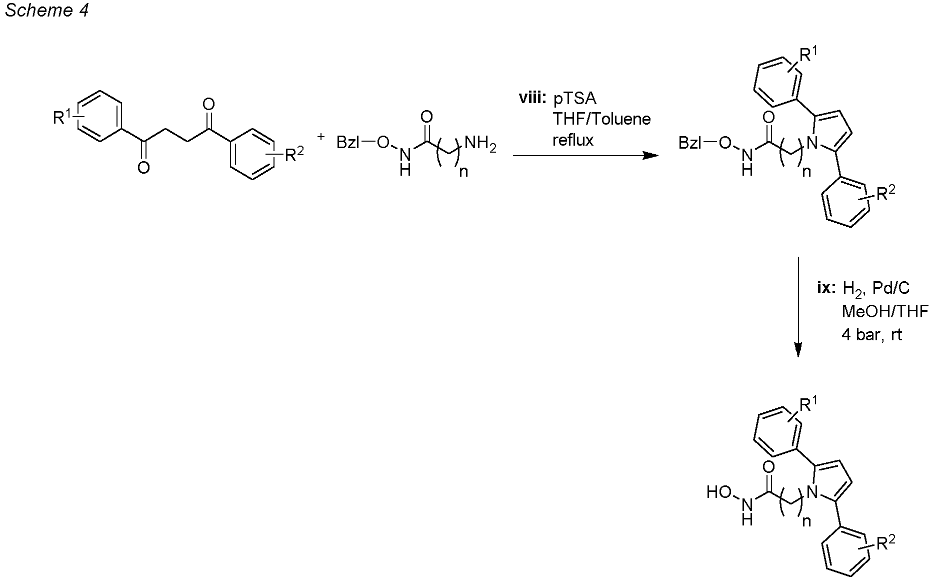

### Method viii:

The respective 1,4-butanedione (1 eq) was dissolved in THF/Toluene (1:1 v/v, c=0.1 M). A suitable benzyl-protected amino acid derivative (1.5 eq) and para-toluenesulfonic acid (0.07 eq) were added and the mixture was heated to reflux for 48 hours. The volatiles were evaporated, and the remains were re-dissolved in water, slightly basified by means of aqueous NaHCO₃ and extracted with EtOAc (3x25 ml). The combined organic layers were evaporated and purified by flash chromatography (silica, heptane-EtOAc gradient).

### Method ix:

The respective benzyl-protected hydroxamic acid derivative obtained by method viii was dissolved in MeOH/THF (1:1 v/v, 10 ml). Palladium on charcoal was added and the vial was purged with hydrogen. After 4 hours at 4 bar, the mixture was filtered through Celite and evaporated. The residue was purified by semi-preparative HPLC.

### Example 9: 2-(2,5-Diphenylpyrrol-1-yl)ethanehydroxamic acid

The compound was synthesized according to methods viii and ix as described above. Yield (last step): 25 mg (37%); ESI-MS: m/z 293.2 [M+H]⁺; HPLC (gradient 1): rt 15.84 min (97.7%); ¹H NMR, 400 MHz, DMSO d₆: δ 4.34 (s, 1.5H), 4.65 (s, 0.5H), 6.27 (s, 2H), 7.33-7.36 (m, 2H), 7.41-7.47 (m, 8H), 8.99 ( br s, 0.6H), 9.19 (br s, 0.2H), 10.26 (br s, 0.2H), 10.54 (br s, 0.8H) mixture of cis-trans isomers.

### Example 10: 3-(2,5-Diphenylpyrrol-1-yl)propanehydroxamic acid

The compound was synthesized according to methods viii and ix as described above. Yield (last step): 9 mg (4%); ESI-MS: m/z 307.4 [M+H]⁺, 329.4 [M+Na]⁺; HPLC (gradient 1): rt 16.27 min (>99%); ¹H NMR, 400 MHz, DMSO d₆: δ 1.83-1.87 (m, 1.8H), 2.09-2.13 (m, 0.2H), 4.28-4.32 (m, 2H), 6.23 (s, 2H), 7.34-7.38 (m, 2H), 7.44-7.51 (m, 8H), 8.57 (br s, 0.6H), 8.81 (br s, 0.1H), 9.76 (br s, 0.1H), 10.16 (br s, 0.9H) mixture of cis-trans isomers.

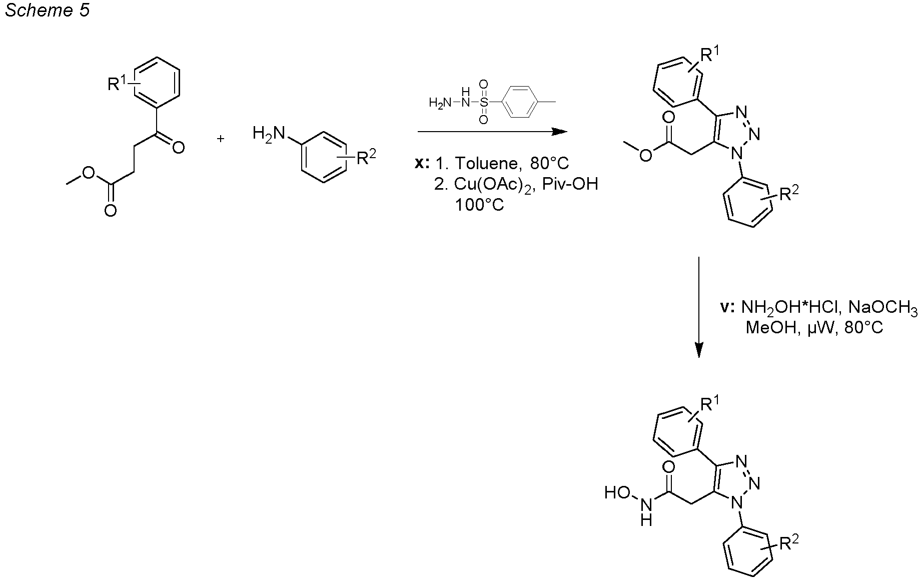

### Method x:

The respective 4-oxo-4-phenylbutanoate (1 eq) was dissolved in toluene (c=0.2 M). Tosylhydrazide (1 eq) was added and the mixture was stirred at 80°C for 2 hours. The aniline derivative (2 eq), Cu(OAc)₂ (1 eq) and pivalic acid (2 eq) were added and the mixture was heated to 100°C overnight. The volatiles were evaporated, and the residue was purified by flash chromatography (silica, heptane/diethylether gradient).

### Example 11: 2-(3,5-Diphenyltriazol-4-yl)ethanehydroxamic acid

The compound was synthesized according to methods x and v as described above. Yield (last step): 23 mg (32%); ESI-MS: m/z 295.1 [M+H]⁺; HPLC (gradient 1): rt 10.43 min (>99%); ¹H NMR, 400 MHz, DMSO d₆: δ 3.63 (s, 1.7H), 3.92 (s, 0.3H), 7.42-7.45 (m, 1H), 7.52 (t, 2H, ³*J* = 7.8 Hz), 7.57-7.69 (m, 5H), 7.75-7.77 (m, 2H), 9.04 (br s, 0.6H), 9.39 (br s, 0.2H), 10.33 (s, 0.1H), 10.76 (s, 0.9H) mixture of cis-trans isomers.

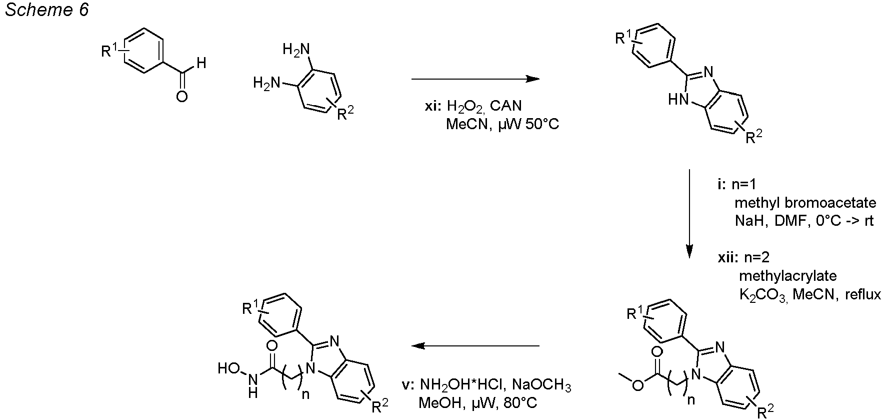

### Method xi:

Ortho-phenylendiamine, or a substituted analogue, (1 eq) was dissolved in acetonitrile (c=0.2 M). The respective aldehyde (1 eq), 30% hydrogen peroxide (4 eq) and ceric ammonium nitrate (0.1 eq) were added and the mixture was heated to 50°C for 12 minutes under microwave irradiation. The volatiles were evaporated, the remains were taken up with water and extracted with EtOAc (3x25 ml). The combined organic layers were dried over Na₂SO₄ and evaporated. The residue was purified by flash chromatography (silica, CHCl₃/MeOH gradient).

### Method xii:

The respective 2-phenylbenzimimidazole obtained by method xi (1 eq) was dissolved in acetonitrile (2 ml). Methyl acrylate (1.1 eq) and K₂CO₃ (1 eq) were added and the mixture was heated under reflux for 6 hours. The volatiles were evaporated and taken up with a small amount of EtOAc. Water was added, and the mixture was extracted with EtOAc (3x25 ml). The combined organic layers were dried over Na₂SO₄ and evaporated. The residue was purified by flash chromatography (silica, heptane/EtOAc gradient).

### Example 12: 2-(2-Phenylbenzimidazol-1-yl)ethanehydroxamic acid

The compound was synthesized according to methods xi, i and v as described above. Yield (last step): 88 mg (66%); ESI-MS: m/z 268.3 [M+H]⁺; HPLC (gradient 1): rt 5.96 min (>99%); ¹H NMR, 400 MHz, DMSO d₆: δ 4.95 (s, 1.6 H), 5.29 (s, 0.4 H), 7.44-7.51 (m, 2H), 7.64-7.71 (m, 4H), 7.78-7.84 (m, 1H), 7.88-7.90 (m, 2H), 10.62 (br s, 0.2 H), 11.10 (br s, 0.8 H) mixture of cis-trans isomers.

### Example 13: 2-[2-(4-Chloro-2-fluoro-3-hydroxy-phenyl)benz-imidazol-1-yl]ethanehydroxamic acid

The compound was synthesized according to methods xi, i and v as described above, final deprotection of the phenol was accomplished by treatment with BBr₃(1 M in DCM, 5 eq). Yield (last step): 24 mg (51%); ESI-MS: m/z 336.5 [M+H]⁺; HPLC (gradient 1): rt 7.31 min (>99%); ¹H NMR, 400 MHz, DMSO d₆: δ 4.80 (s, 1.6H), 5.13 (s, 0.4H), 7.06-7.46 (m, 3H), 7.62 (d, 1H, ³*J* = 7.7 Hz), 7.77 (d, 1H, ³*J* = 7.7 Hz), 10.43 (br s, 0.2 H), 10.91 (br s, 0.8H) mixture of cis-trans isomers.

### Example 14: 3-(2-phenylbenzimidazol-1-yl)propanehydroxamic acid

The compound was synthesized according to methods xi, xii and v as described above. Yield (last step): 33 mg (37%); ESI-MS: m/z 282.4 [M+H]⁺; HPLC (gradient 1): rt 6.61 min (95.7%); ¹H NMR, 400 MHz, DMSO d₆: δ 2.59 (t, 2H, ³*J* = 7.4 Hz), 4.60 (t, 2H, ³*J* = 7.7 Hz), 7.49-7.57 (m, 2H), 7.67-7.74 (m, 3H), 7.80-7.83 (m, 1H), 7.87-7.89 (m, 2H), 7.93-7.95 (m, 1H), 10.05 (br s, 0.1H), 10.50 (br s, 0.9H) mixture of cis-trans isomers.

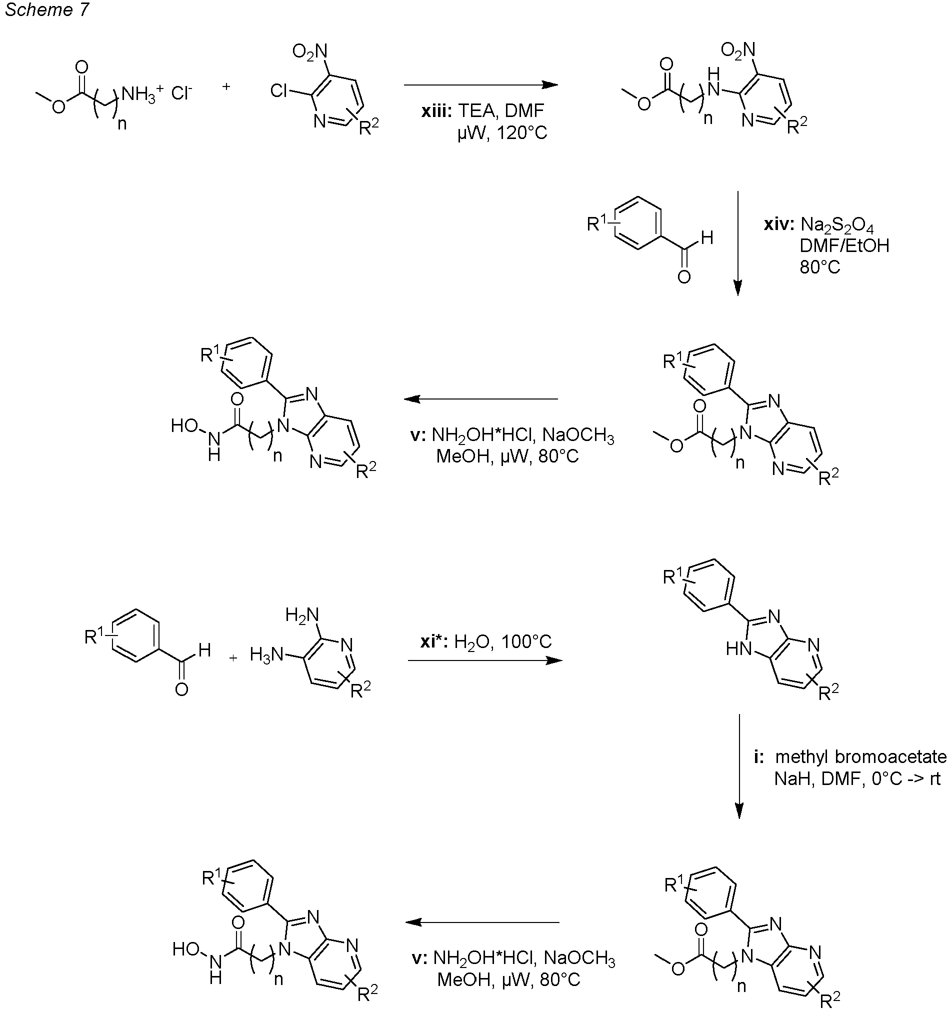

### Method xi*:

Pyridine-2,3-diamine or a substituted analogue (1 eq) was dissolved in water (1 M), treated with the respective aldehyde (1 eq) and heated to reflux overnight. After cooling the mixture was basified with aqueous NaHCO₃ and extracted with EtOAc (3x 25 ml). The combined organic layers were dried over Na₂SO₄ and evaporated. The residue was purified by flash chromatography (silica, CHCl₃/MeOH gradient).

### Method xiii:

The respective amino acid ester (1 eq) was dissolved in dimethylformamide (c=1.0 M). 2-Chloro-3-nitropyridine derivative (1 eq) and triethylamine (2.5 eq) were added and the mixture was heated in a microwave to 120°C for 20 min. After cooling, water was added and extracted with EtOAc (3x25 ml). The combined organic layers were dried over Na₂SO₄ and evaporated. The residue was used without further purification.

### Method xiv:

The nitropyridine derivative obtained by method xiii was dissolved in DMF/EtOH (1:1 v/v, c=0.15M). The respective aldehyde (1 eq) and Na₂S₂O₄ (3 eq) were added and the mixture was stirred at 80°C for 22h. The volatiles were evaporated, and the remains were taken up with a small amount of water. The mixture was extracted with EtOAc (3x25 ml). The combined organic layers were dried over Na₂SO₄ and evaporated. The residue was purified by flash chromatography (silica, heptane/EtOAc 1:1)

### Example 15: 2-(2-Phenylimidazo[4,5-b]pyridin-3-yl)ethane-hydroxamic acid

The compound was synthesized according to methods xiii, xiv and v as described above. Yield (last step): 35 mg (39%); ESI-MS: m/z 269.1 [M+H]⁺; HPLC (gradient 1): rt 6.75 min (>99%); ¹H NMR, 400 MHz, DMSO d₆: δ 4.92 (s, 1.6H), 5.26 (s, 0.4H), 7.36-7.39 (m, 1H), 7.58-7.61 (m, 3H), 7.78-7.80 (m, 0.5H), 7.87-7.89 (m, 1.5H), 8.14-8.16 (dd, 1H, ³*J* = 7.8 Hz, ⁴J=1.2 Hz), 8.38-8.89 (m, 1H), 9.53 (br s, 0.2H), 10.45 (s, 0.2H), 11.01 (s, 0.8H) mixture of cis-trans isomers.

### Example 16: 2-(2-phenylimidazo[4,5-b]pyridin-1-yl)ethane-hydroxamic acid

The compound was synthesized according to methods xi*, i and v as described above. Yield (last step): 38 mg (20%); ESI-MS: m/z 269.1 [M+H]⁺; HPLC (gradient 1): rt 6.61 min (>99%); ¹H NMR, 400 MHz, DMSO d₆: δ 5.54 (s, 1.4H), 5.86 (s, 0.6H), 7.69-7.71 (m, 3H), 7.98 (t, 1H, ³*J* = 7.1 Hz), 8.32-8.34 (m, 2H), 8.73-8.79 (m, 2H), 9.32 (br s, 0.7H), 9.84 (br s, 0.3H), 10.80 (s, 0.3H), 11.27 (s, 0.7H) mixture of cis-trans isomers.

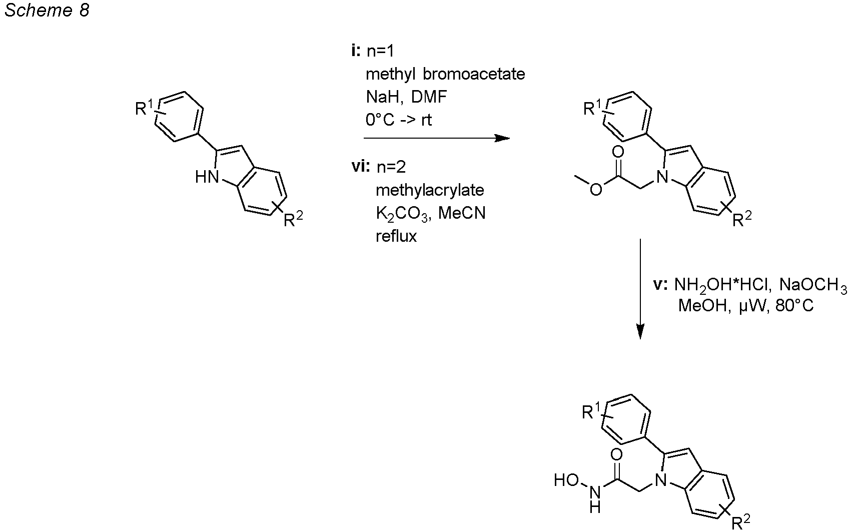

### Example 17: 2-(2-Phenylindol-1-yl)ethanehydroxamic acid

The compound was synthesized according to methods i and v as described above. Yield (last step): 91 mg (34%); ESI-MS: m/z 267.3 [M+H]⁺; HPLC (gradient 1): rt 15.81 min (>99%); ¹H NMR, 400 MHz, DMSO d₆: δ 4.66 (s, 1.7H), 4.99 (s, 0.3H), 6.58 (s,1H), 7.08-7.12 (m, 1H), 7.16-7.20 (m, 1H), 7.36-7.38 (m, 1H), 7.43-7.47 (m, 1H), 7.49-7.53 (m, 2H), 7.58-7.64 (m, 3H), 10.34 (s, 0.1H), 10.93 (s, 0.9H) mixture of cis-trans isomers.

### Example 18: 3-(2-Phenylindol-1-yl)propanehydroxamic acid

The compound was synthesized according to methods vi and v as described above. Yield (last step): 33 mg (18%); ESI-MS: m/z 281.2 [M+H]⁺; HPLC (gradient 1): rt 15.22 min (>99%); ¹H NMR, 400 MHz, DMSO d₆: δ 2.34-2.38 (m, 2H), 4.36-4.40 (m, 2H), 6.54 (s, 1H), 7.07-7.10 (m, 1H), 7.18-7.22 (m, 1H), 7.45-7.49 (m, 1H), 7.51-7.58 (m, 6H), 9.97 (br s, 0.1H), 10.46 (br s, 0.9H) mixture of cis-trans isomers.

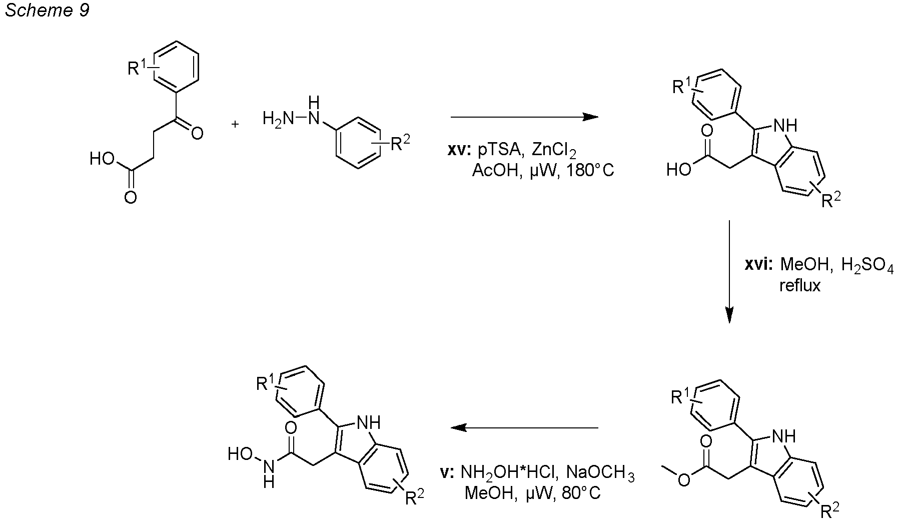

### Method xv:

The respective benzoylpropionic acid (1 eq) was dissolved in acetic acid (c=0.33 M). Phenylhydrazine (1.2 eq), para-toluenesulfonic acid (1.1 eq) and ZnCl₂ (1 eq) were added and the mixture was heated to 180°C in a microwave for 40 minutes. After cooling, water was added, and the mixture was extracted with EtOAc (3x25 ml). The combined organic layers were dried over Na₂SO₄ and evaporated. The residue was purified by flash chromatography (silica, heptane-EtOAc gradient).

### Method xvi:

The respective indol derivative obtained by method xv was dissolved in methanol (5 ml) and treated with conc. H₂SO₄. The mixture was heated to reflux for 4 hours. The volatiles were evaporated, re-dissolved in dichloromethane and washed carefully with saturated aq. NaHCO₃. The organic layer was dried over Na₂SO₄ and evaporated. The residue was used without further purification.

### Example 19: 2-(2-Phenyl-1H-indol-3-yl)ethanehydroxamic acid

The compound was synthesized according to methods xv, xvi and v as described above. Yield (last step): 14 mg (15%); ESI-MS: m/z 267.2 [M+H]⁺, 206.1 [M-CH₂NO₂]⁺; HPLC (gradient 1): rt 11.97 min (>99%); ¹H NMR, 400 MHz, DMSO d₆: δ 3.51 (s, 1.9H), 3.85 (s, 0.1H), 7.02 (t, 1H, ³*J* = 7.6 Hz), 7.12 (t, 1H, ³*J* = 7.5 Hz), 7.36-7.42 (m, 2H), 7.51 (t, 2H, ³*J* = 7.7 Hz), 7.61 (d, 1H, ³*J* = 7.8 Hz), 7.87 (d, 2H, ³*J* = 7.8 Hz), 10.77 (s, 1H), 11.26 (s, 1H) mixture of cis-trans isomers.

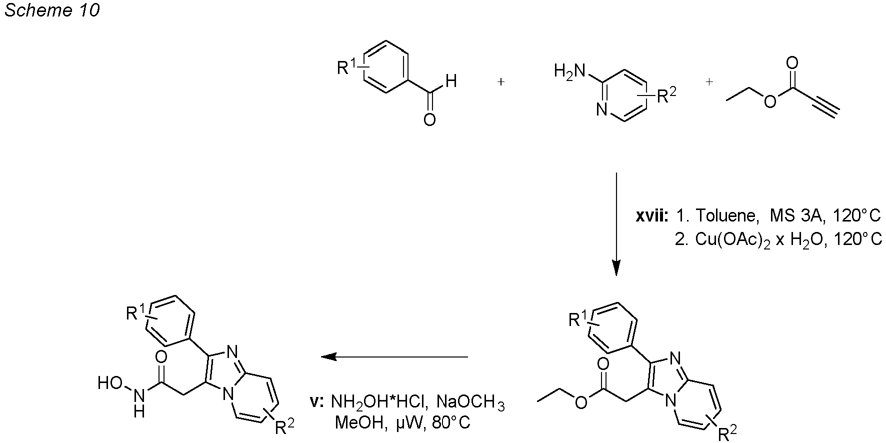

### Method xvii:

Aminopyridine (1 eq) and the respective aldehyde (1.2 eq) were dissolved in toluene (c=0.2 M). Molecular sieves (3Å, 200 mg) were added and the mixture was heated to 120°C for 14 hours. After cooling to room temperature, Cu(OAc)₂ x H₂O (0.1 eq) and ethyl propiolate (1 eq) were added and heated to 120°C for 3 hours. Afterwards, the mixture was filtered through Celite and the volatiles were evaporated. The residue was purified by flash chromatography (silica, CHCl₃-MeOH gradient).

### Example 20: 2-[2-(4-Chloro-2-fluoro-3-hydroxy-phenyl)imidazo-[1,2-a]pyridin-3-yl]ethanehydroxamic acid

The compound was synthesized according to methods xvii and v as described above, final deprotection of the phenol was accomplished by treatment with BBr₃ (1M in DCM, 5 eq). Yield (last step): 7 mg (13%); ESI-MS: m/z 336.2 [M+H]⁺; HPLC (gradient 1): rt 6.75 min (>99%); ¹H NMR, 400 MHz, DMSO d₆: δ 3.90 (s, 1.7H), 4.19 (s, 0.3H), 7.05-7.14 (m, 1H), 7.42-7.44 (m, 2H), 7.81-7.93 (m, 2H), 8.62-8.74 (m, 1H), 9.01 (br s, 0.3H), 9.45 (br s, 0.1H), 10.37 und 10.84 (br s, 1H), 10.80 (s, 1H) mixture of cis-trans isomers.

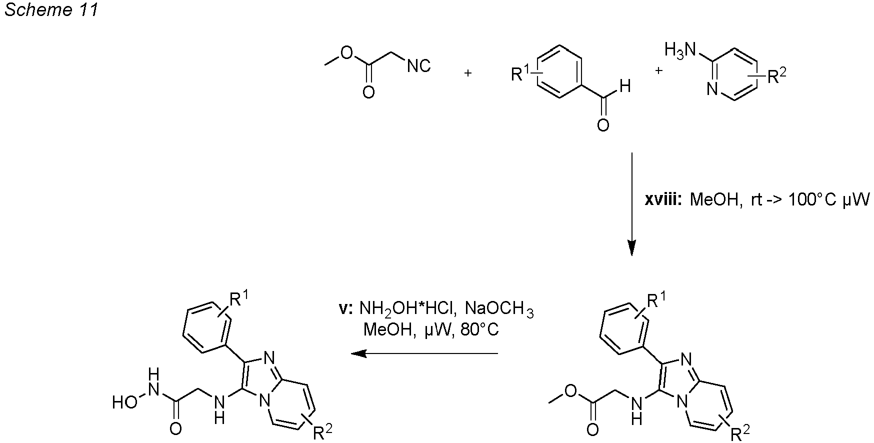

### Method xviii:

2-Aminopyridine, or a substituted analogue, (1 eq) was dissolved in MeOH (c=0.4 M). The respective aldehyde (1 eq) was added and the mixture was stirred at ambient temperature for 10 minutes. After addition of methyl isocyanoacetate (1 eq), the reaction was heated at 100°C for 30 minutes under microwave irradiation. After cooling, cold diethylether was added. The resulting precipitate was collected by filtration and used without further purification.

### Example 21: 2-[(2-Phenylimidazo[1,2-a]pyridin-3-yl)amino]-ethanehydroxamic acid

The compound was synthesized according to methods xviii and v as described above. Yield (last step): 32 mg (6%); ESI-MS: m/z 283.2 [M+H]⁺; HPLC (gradient 2): rt 6.91 min (>99%); ¹H NMR, 400 MHz, DMSO d₆: δ 3.56 (s, 1.8H), 3.90 (s, 0.2H), 5.86 (br s, 1H), 7.44-7.53 (m, 2H), 7.61 (t, 2H, ³*J* = 7.8 Hz), 7.83-7.84 (m, 2H), 8.01-8.03 (m, 2H), 8.91-8.93 (m, 2H), 10.10 (br s, 0.3H), 10.54 (br s, 0.7H) mixture of cis-trans isomers.

### Example 22: 4-[3-[[2-(Hydroxyamino)-2-oxo-ethyl]amino]-imidazo[1,2-a]pyridin-2-yl]benzoic acid

The compound was synthesized according to methods xviii and v as described above. Yield (last step): 67 mg (11%); ESI-MS: m/z 327.2 [M+H]⁺; HPLC (gradient 2): rt 6.69 min (93.4%); ¹H NMR, 400 MHz, DMSO d₆: δ 3.91 (s, 2H), 5.92 (br s, 1H), 7.35-7.41 (m, 1H), 7.71-7.79 (m, 2H), 8.11-8.17 (m, 4H), 8.84-8.90 (m, 2H), 10.51 (s, 1H), 13.13 (br s, 1H).

### Example 23: 3-[3-[[2-(Hydroxyamino)-2-oxo-ethyl]amino]-imidazo[1,2-a]pyridin-2-yl]benzoic acid

The compound was synthesized according to methods xviii and v as described above. Yield (last step): 39 mg (10%); ESI-MS: m/z 327.2 [M+H]⁺; HPLC (gradient 2): rt 7.15 min (98.9%); ¹H NMR, 400 MHz, DMSO d₆: δ 3.90 (s, 2H), 5.93 (br s, 1H), 7.41-7.45 (m, 1H), 7.72 (t, 1H, ³*J* = 7.8 Hz), 7.80-7.84 (m, 2H), 8.03-8.05 (m, 1H), 8.29-8.31 (m, 1H), 8.59 (br s, 1H), 8.89-8.96 (m, 2H), 10.52 (br s, 1H).

### Example 24: 2-[[2-(2,3-Dihydro-1,4-benzodioxin-6-yl)imidazo-[1,2-a]pyridin-3-yllamino]ethanehydroxamic acid

The compound was synthesized according to methods xviii and v as described above. Yield (last step): 36 mg (10%); ESI-MS: m/z 327.2 [M+H]⁺; HPLC (gradient 2): rt 8.19 min (96.8%); ¹H NMR, 400 MHz, DMSO d₆: δ 3.55 (s, 2H), 4.34 (s, 4H), 5.81 (br s, 1H), 7.09-7.11 (m, 1H), 7.46-7.55 (m, 3H), 7.81-7.88 (m, 2H), 8.92-8.94 (m, 2H), 10.11 (br s, 0.3H), 10.53 (br s, 0.7H) mixture of cis-trans isomers.

### Example 25: 3-[[2-(Hydroxyamino)-2-oxo-ethyl]amino]-2-phenyl-imidazo[1,2-a]pyridine-8-carboxylic acid

The compound was synthesized according to methods xviii and v as described above. Yield (last step): 16 mg (11%); ESI-MS: m/z 327.4 [M+H]⁺; HPLC (gradient 1): rt 5.81 min (98.0%); ¹H NMR, 400 MHz, DMSO d₆: δ 3.51 (s, 2H), 5.78 (br s, 1H), 7.36-7.63 (m, 6H), 7.98-8.04 (m, 2H), 8.17-8.29 (m, 1H), 10.50 (s, 1H).

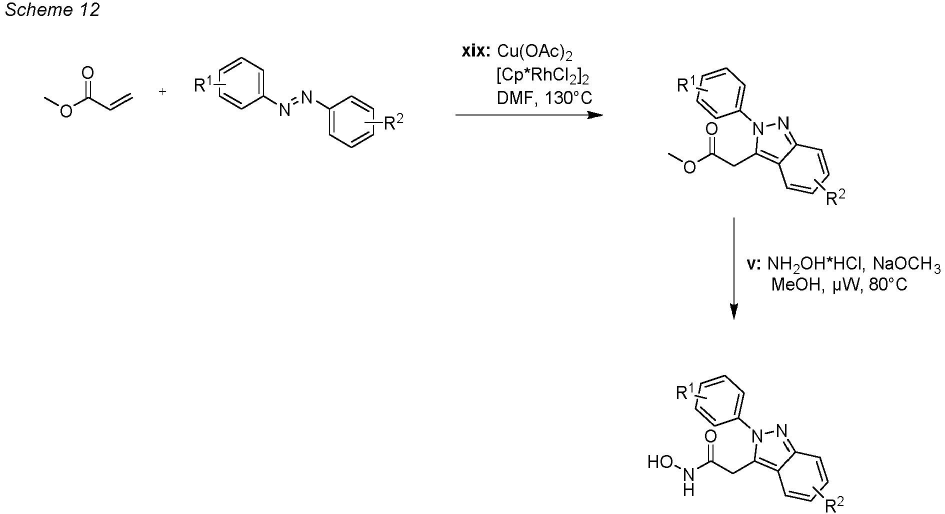

### Method xix:

A vial containing the respective azabenzene (1 eq), [Cp*RhCl₂]₂ (0.05 eq) and Cu(OAc)₂ (2 eq) was sealed and purged with argon. Dimethylformamide (c=0.2 M) and methyl acrylate (1.2 eq) were added and the mixture was stirred at 130°C overnight. The reaction was quenched with water and extracted with EtOAc (3x25 ml). The combined organic layers were dried over Na₂SO₄ and evaporated. The residue was purified by flash chromatography (silica, heptane-EtOAc gradient).

### Example 26: 2-(2-Phenylindazol-3-yl)ethanehydroxamic acid

The compound was synthesized according to methods xix and v as described above. Yield (last step): 18 mg (56%); ESI-MS: m/z 268.1 [M+H]⁺; HPLC (gradient 1): rt 9.49 min (>99%); ¹H NMR, 400 MHz, DMSO d₆: δ 3.83 (s, 1.8H), 4.15 (s, 0.2H), 7.08-7.11 (m, 1H), 7.30-7.34 (m, 1H), 7.55-7.66 (m, 4H), 7.72-7.78 (m, 3H), 10.21 (s, 0.1H), 10.86 (s, 0.9H) mixture of cis-trans isomers.

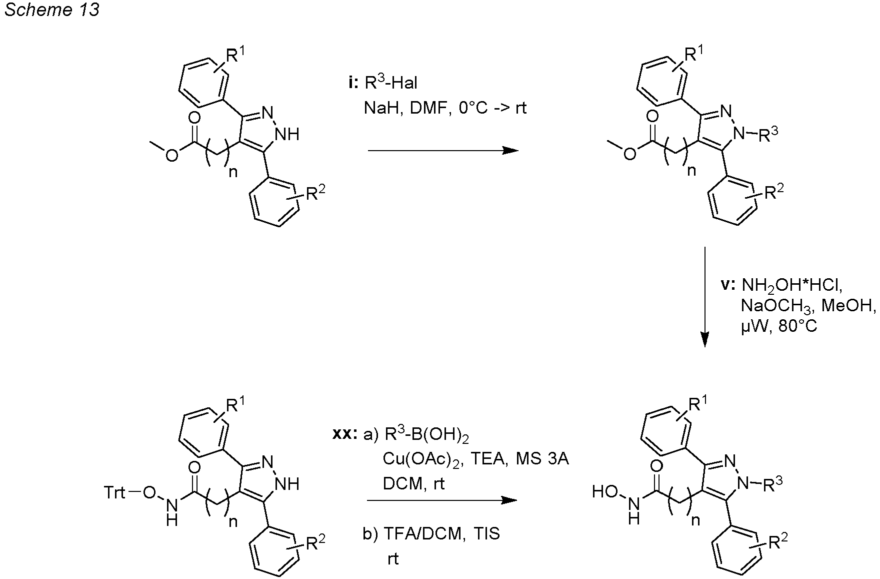

### Method xx:

2-(3,5-Diphenyl-1H-pyrazol-4-yl)-N-trityloxy-acetamide (1 eq) was dissolved in dichloromethane (c=0.1 M). The respective boronic acid (2 eq), triethylamine (2 eq), Cu(OAc)₂ (1.5 eq) and molecular sieves were added, and the mixture was vigorously stirred at room temperature until TLC showed full conversion of the starting materials. The mixture was filtered through celite and evaporated. The residue was purified by flash chromatography (silica, heptane-EtOAc gradient). The purified product was treated with TFA/DCM (1:1 v/v, 10 ml) and triisopropylsilane (180 µl) and stirred at room temperature for 1-2 hours. The volatiles were evaporated, and the remains were purified by semi-preparative HPLC.

### Example 27: 2-(1-Methyl-3,5-diphenyl-pyrazol-4-yl)ethane-hydroxamic acid

The compound was synthesized according to methods i and v as described above. Yield (last step): 96 mg (45%); ESI-MS: m/z 308.2 [M+H]⁺; HPLC (gradient 1): rt 11.55 min (>99%); ¹H NMR, 400 MHz, DMSO d₆: δ 3.14 (s, 1.8H), 3.45 (s, 0.2H), 3.77 (s, 3H), 7.33-7.37 (m, 1H), 7.41-7.58 (m, 7H), 7.64-7.67 (m, 2H), 10.00 (s, 0.1H), 10.45 (s, 0.9H) mixture of cis-trans isomers.

### Example 28: 2-(1-Benzyl-3,5-diphenyl-pyrazol-4-yl)ethane-hydroxamic acid

The compound was synthesized according to methods i and v as described above. Yield (last step): 81 mg (35%); ESI-MS: m/z 383.4 [M+H]⁺; HPLC (gradient 1): rt 15.63 min (>99%); ¹H NMR, 400 MHz, DMSO d₆: δ 3.18 (s, 1.8H), 3.47 (s, 0.2H), 5.28 (s, 2H), 7.01-7.04 (m, 2H), 7.22-7.31 (m, 3H), 7.34-7.52 (m, 8H), 7.58-7.70 (m, 2H), 9.97 (s, 0.1H), 10.45 (S, 0.9H) mixture of cis-trans isomers.

### Example 29: 2-(1,3,5-triphenylpyrazol-4-yl)ethanehydroxamic acid

The compound was synthesized according to methods xx as described above. Yield (last step): 50 mg (14%); ESI-MS: m/z 370.3 [M+H]⁺; HPLC (gradient 1): rt 15.63 min (>99%); ¹H NMR, 400 MHz, DMSO d₆: δ 3.24 (s, 1.8H), 3.54 (s, 0.2H), 7.28-7.50 (m, 13H), 7.66-7.77 (m, 2H), 10.06 (s, 0.1H), 10.53 (s, 0.9H) mixture of cis-trans isomers.

### Example 30: 4-[4-[2-(Hydroxyamino)-2-oxo-ethyl]-3,5-diphenyl-pyrazol-1-yl]benzoic acid

The compound was synthesized according to methods i and v as described above. Yield (last step): 55 mg (13%); ESI-MS: m/z 414.2 [M+H]⁺; HPLC (gradient 1): rt 13.08 min (>99%); ¹H NMR, 400 MHz, DMSO d₆: δ 3.24 (s, 1.8H), 3.55 (s, 0.2H), 7.28-7.52 (m, 10H), 7.68-7.79 (m, 2H), 7.90-7.92 (m, 2H), 10.09 (s, 0.1H), 10.55 (s, 0.9H), 13.06 (br s, 1H) mixture of cis-trans isomers.

### Example 31: 3-[4-[2-(Hydroxyamino)-2-oxo-ethyl]-3,5-diphenyl-pyrazol-1-yl]benzoic acid

The compound was synthesized according to methods i and v as described above. Yield (last step): 45 mg (11%); ESI-MS: m/z 414.3 [M+H]⁺; HPLC (gradient 1): rt 13.09 min (>99%); ¹H NMR, 400 MHz, DMSO d₆: δ 3.25 (s, 1.8H), 3.55 (s, 0.2H), 7.27-7.51 (m, 10H), 7.67-7.78 (m, 2H), 7.83-7.91 (m, 2H), 10.08 (s, 0.1H), 10.53 (s, 0.9H), 13.13 (br s, 1H) mixture of cis-trans isomers.

### Analytical methods

**HPLC:** The analytical HPLC-system consisted of a Merck-Hitachi device (model LaChrom) utilizing a LUNA RP 18 (5 µm), analytical column (length: 125 mm, diameter: 4 mm), and a diode array detector (DAD) with λ = 214 nm as the reporting wavelength. The compounds were analysed using a gradient at a flow rate of 1 mL/min; whereby eluent (A) was acetonitrile, eluent (B) was water, both containing 0.04 % (v/v) trifluoroacetic acid applying one of the following gradients:
*Gradient 1:* 0 min - 5 min -> 5% (A), 5 min - 15 min -> 5 - 60% (A), 15 min - 20 min 60 - 95% (A) 20 min - 30 min 95% (A)
*Gradient 2:* 0 min - 15 min 5 - 50 % (A), 15 min - 20 min -> 50 - 95 % (A), 20 min - 23 min 95 % (A)

The purities of all reported compounds were determined by the percentage of the peak area at 214 nm.

**Mass**-**spectrometry, NMR-spectroscopy:** ESI-Mass spectra were obtained with a SCIEX API 1200 spectrometer (Perkin Elmer) or an expressionCMS (Advion). The ¹H NMR-Spectra were recorded at an Agilent DD2 400-MHz spectrometer. Chemical shifts (δ) are expressed as parts per million (ppm) downfield from tetramethylsilane. Splitting patterns have been designated as follows: s (singlet), d (doublet), dd (doublet of doublet), t (triplet), m (multiplet) and br (broad signal).

### II. Enzymatic assays

The determination of enzymatic activity was based on the cleavage of internally quenched peptide substrates. A typical assay of 250 µl total volume measured in black 96 well plates consisted of 100 µl buffer, 50 µl enzyme at a final concentration of 5e-8 M to 5e-9 M, 50 µl substrate (0.15 to 80 µM, in buffer, 0.5% DMSO) and 50 µl inhibitor solution (in buffer, 1% DMSO). In case of 125 µl assay volume (black 96 half area well plates) all volumes were cut in half. Enzymatic activity of ADAMs was measured in 384 well plates with 60 µl total assay volume consisting of 20 µl inhibitor, 20 µl buffer, 10 µl enzyme and 10 µl substrate.

**Table 1. Peptide substrates and assay conditions used for determination of enzymatic activity (Abz = 2-aminobenzoyl; Dnp = 2,4-dinitrophenyl; Mca = 7-methoxy coumarin; Dap = 2,3-diaminopropionic acid; hMeprin = human meprin; hMMPs = human Matrix Metalloproteases, hADAMs = human A Desintegrin and Metalloproteases)**

| Enzyme | Substrate | Buffer | Assay volume |
|---|---|---|---|
| hMeprin β | Abz-YVAEAPK(Dnp)G-OH | 40 mM Tris pH 8.0 | 250 µl |
| hMeprin α | Abz-YVADAPK(Dnp)G-OH | 40 mM HEPES pH 7.4, 100 mM NaCl | 250 µl |
| hMMP 2 (Abnova) | Mca-PLGL-(DapDnp)-AR-NH₂ | 50 mM Tris, 2 µM ZnCl₂, 150 mM NaCl, pH 8.5 | 125 µl |
| hMMPs 9 and 13 (R&D systems) | Mca-PLGL-(DapDnp)-AR-NH₂ | 50 mM Tris, 2 µM ZnCl₂, 150 mM NaCl, pH 7.5 | 125 µl |
| hADAMs 10 and 17 (R&D systems) | Abz-LANAVRSSSR-(DapDnp)-NH₂ | 25 mM Tris, 2 µM ZnCl₂, 150 mM NaCl, pH 9.0 | 60 µl |

For IC₅₀ values, the influence of 12 inhibitor concentrations ranging from 0 to 5e-5 M on the enzymatic activity was investigated in the presence of one standard substrate concentration (10 µM). Initial velocities were determined and converted into concentration units applying a standard curve obtained after complete conversion of different substrate concentrations under assay conditions. All measurements were performed using a fluorescence plate reader (FLUOstar OPTIMA, BMG Labtech) at 30°C. The kinetic parameters were determined at least in duplicates on separate days. The excitation/emission wavelength was 340/420 nm. The kinetic data was evaluated using GraFit software (version 7.0.3, Erithacus Software).

MMPs were activated prior to measurement by APMA (p-aminophenylmercuric acetate) treatment according to manufacturer's instructions (R&D systems).

### III. Inhibition of Meprin α and β

The following compounds according to the present invention were synthesized using the above general procedures. IC₅₀ values for the inhibition of hMeprin β and α measured using the above enzyme assays are shown in the following table. IC₅₀ refers to the average IC₅₀ values (geometric mean of independent experiments; geometric SD factor is given in parentheses) measured as described above.

**Table 2. Compounds and Inhibitory Activities against Meprin α and β**

| Structure | Compound ID | Meprin alpha | | Meprin beta | |
|---|---|---|---|---|---|
| | | IC₅₀ [nM] | Average pIC₅₀ | IC₅₀ [nM] | Average pIC₅₀ |
| 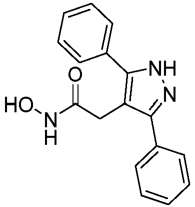 | Example 1 | 4 (1.05) | 8.37 | 204 (1.06) | 6.69 |
| 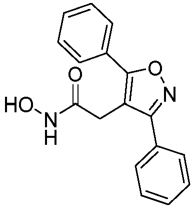 | Example 2 | 7 (1.05) | 8.13 | 831 (1.12) | 6.08 |
| 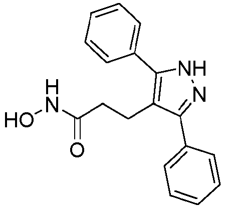 | Example 3 | 108 (1.02) | 6.96 | 8075 (1.07) | 5.09 |
| 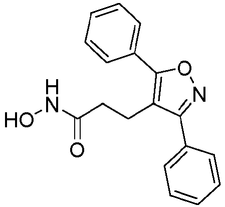 | Example 4 | 32 (1.002) | 7.49 | 13691 (1.05) | 4.86 |
| 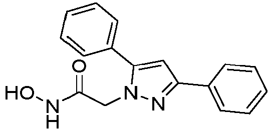 | Example 5 | 292 (1.13) | 6.53 | 10492 (1.06) | 4.98 |
| 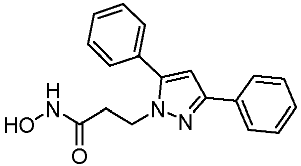 | Example 6 | 457 (1.03) | 6.34 | 15445 (1.13) | 4.81 |
| 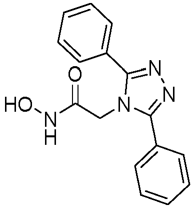 | Example 7 | 69 (1.004) | 7.16 | 8712 (1.04) | 5.06 |
| 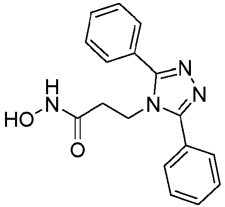 | Example 8 | 353 (1.09) | 6.45 | 42320 (1.09) | 4.37 |
| 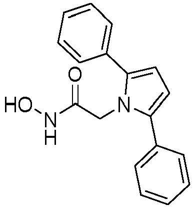 | Example 9 | 34 (1.12) | 7.46 | 4317 (1.09) | 5.36 |
| 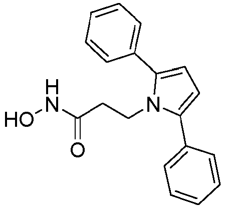 | Example 10 | 727 (1.09) | 6.14 | 15026 (1.08) | 4.82 |
| 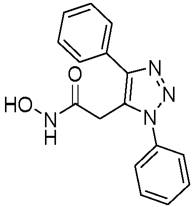 | Example 11 | 11 (1.09) | 7.96 | 1450 (1.03) | 5.84 |
| 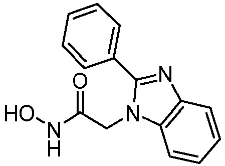 | Example 12 (reference example) | 173 (1.13) | 6.76 | 1628 (1.08) | 5.79 |
| 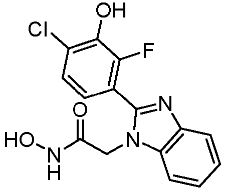 | Example 13 | 199 (1.03) | 7.34 | 46 (1.09) | 6.70 |
| 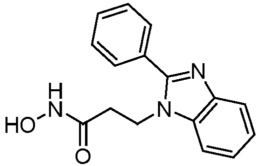 | Example 14 (reference example) | 1130 (1.04) | 5.95 | 28037 (1.04) | 4.55 |
| 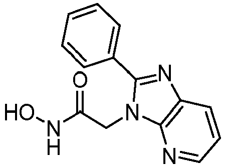 | Example 15 | 273 (1.03) | 6.56 | 4556 (1.12) | 5.34 |
| 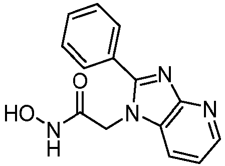 | Example 16 | 1535 (1.29) | 5.81 | 4174 (1.08) | 5.38 |
| 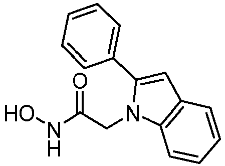 | Example 17 | 72 (1.03) | 7.14 | 1136 (1.13) | 5.94 |
| 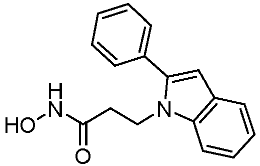 | Example 18 | 475 (1.06) | 6.32 | 13372 (1.17) | 4.87 |
| 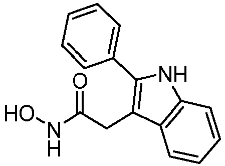 | Example 19 | 105 (1.13) | 6.98 | 1205 (1.04) | 5.92 |
| 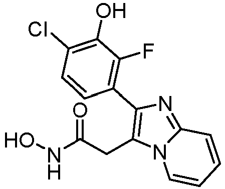 | Example 20 | 135 (1.01) | 6.87 | 23 (1.02) | 7.64 |
| 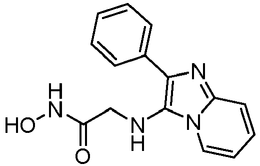 | Example 21 (reference example) | 793 (1.01) | 6.10 | 7117 (1.07) | 5.15 |
| 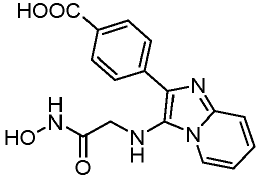 | Example 22 | 2284 (1.18) | 5.64 | 176 (1.004) | 6.76 |
| 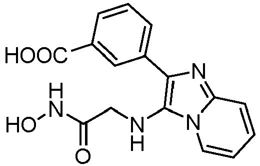 | Example 23 | 693 (1.02) | 6.16 | 214 (1.04) | 6.67 |
| 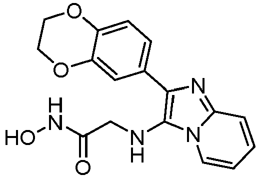 | Example 24 | 655 (1.01) | 6.18 | 3139 (1.04) | 5.50 |
| 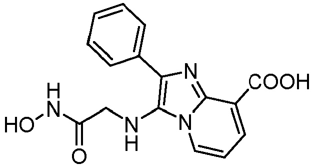 | Example 25 | 6745 (1.005) | 5.17 | 3104 (1.09) | 5.51 |
| 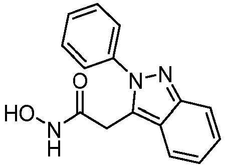 | Example 26 | 244 (1.06) | 6.61 | 3365 (1.08) | 5.47 |
| 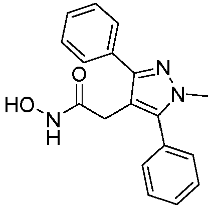 | Example 27 | 9 (1.04) | 8.07 | 1220 (1.00) | 5.91 |
| 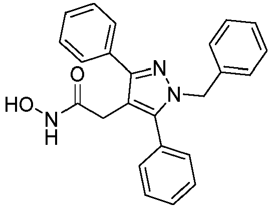 | Example 28 | 2 (1.04) | 8.75 | 254 (1.15) | 6.60 |
| 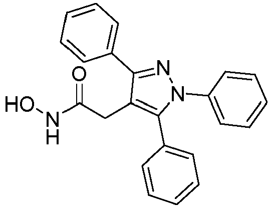 | Example 29 (reference example) | 17 (1.06) | 7.76 | 2378 (1.27) | 5.62 |
| 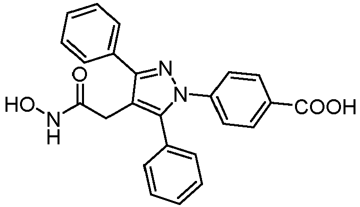 | Example 30 | 21 (1.04) | 7.69 | 154 (1.21) | 6.81 |
| 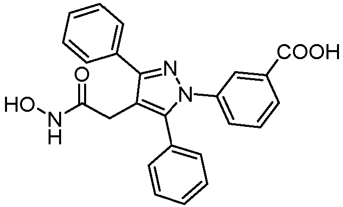 | Example 31 | 16 (1.04) | 7.79 | 100 (1.05) | 7.00 |

### IV. Inhibition of selected other metalloproteases

**Table 3. Residual enzyme activity of other metalloproteases in the presence of 200 µM of selected inhibitor compounds.**

| | Residual enzyme activity [%] @ 200 µM inhibitor | | | | |
|---|---|---|---|---|---|
| Compound ID | MMP2 | MMP9 | MMP13 | ADAM10 | ADAM17 |
| Example 1 | 61 | 27 | 67 | 25 | 32 |
| Example 2 | 34 | 16 | 31 | 26 | 9 |
| Example 3 | 77 | 65 | 68 | 12 | 27 |
| Example 4 | 74 | 39 | 66 | 21 | 24 |
| Example 7 | 70 | 54 | 76 | 83 | 56 |
| Reference Example 12 | 46 | 68 | 72 | 76 | 56 |
| Example 13 | 83 | 50 | 84 | 78 | 82 |
| Example 17 | 41 | 53 | 54 | 68 | 56 |

## Claims

1. A compound according to the following Formula I, 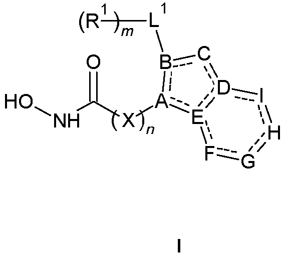 its individual enantiomers, its individual diastereoisomers, its hydrates, its solvates, its crystal forms, its individual tautomers or a pharmaceutically acceptable salt thereof, wherein:
A is independently selected from 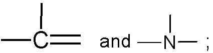
B is independently selected from 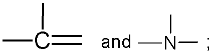
C is independently selected from -N= , 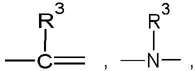 -O- and -S-;
F, if present, is independently selected from 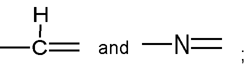
G, if present, is independently selected from 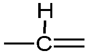 and -N= ;
H, if present, is independently selected from 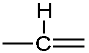 and -N= ;
I, if present, is independently selected from 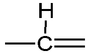 and -N= ;
wherein if F, G, H and I are present, then:
D is 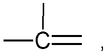
E is s independently elected from 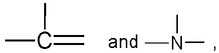 and
the ring formed by D, E, F, G, H and I is substituted by *p* substituents represented by R²,
otherwise if F, G, H and I are absent, then:
D is independently selected from 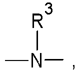 -N= and 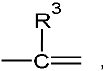 and
E is independently selected from 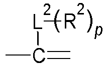 and -N= ,
each X is independently selected form C(R^{a})R^{b}, wherein one of the C(R^{a})R^{b} groups can be replaced by a NR^{a} group ;
*n is* 1 or 2;
*m* is 0, 1, 2 or 3;
*p* is 0, 1, 2, or 3;
L¹ is phenyl;
L² is phenyl;
R¹ is independently selected from Cl, F, OH, OCH₃ and COOH, and/or two R¹ groups together form part of a 1,3-benzodioxol ring or a 2,3-dihydro-1,4-benzodioxin ring;
R² is COOH;
each R³ is independently selected from hydrogen, methyl, ethyl, propargyl, -CH₂COOH, benzyl, unsubstituted phenyl, and substituted phenyl selected from 3-carboxyphenyl and 4-carboxyphenyl; and
R^{a} and R^{b} are hydrogen;
with the provisos that
when the ring fragment 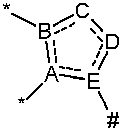 is represented by 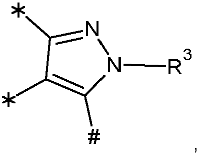 then:
R³ is selected from hydrogen, methyl, benzyl, and substituted phenyl selected from 3-carboxyphenyl and 4-carboxyphenyl;
when the ring fragment 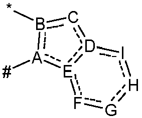 is represented by 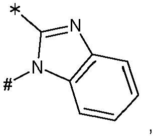 then at least one of *m* and *p* is larger than 0; and
when the ring fragment 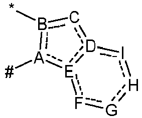 is represented by 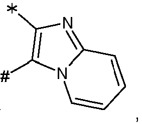 then *m* is larger than 0.

2. The compound according to claim 1, which is represented by one the following Formulae la and lb:

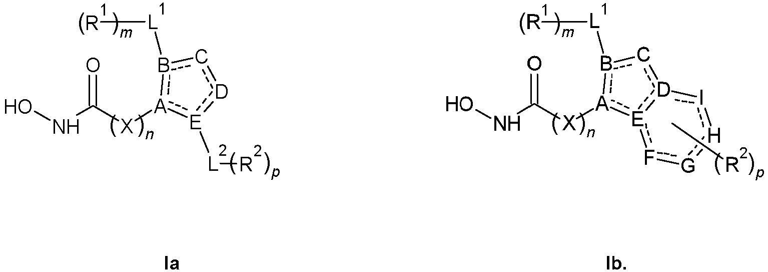

3. The compound according to any one of claims 1 or 2, wherein if F, G, H and I are absent, the ring fragment 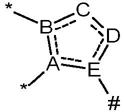 is represented by one of the following structures: 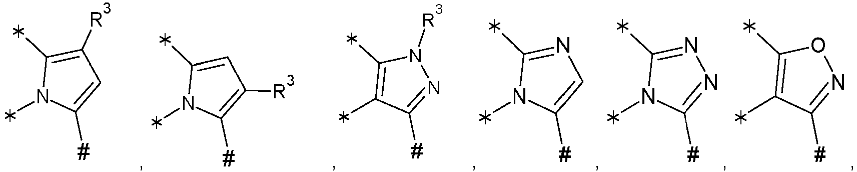 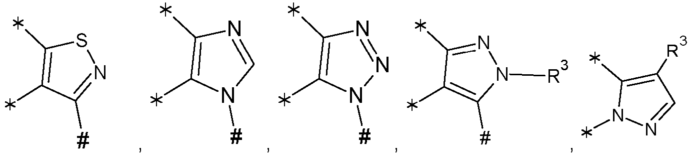 and

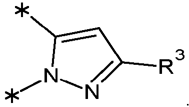

4. The compound according to any one of claims 1 to 3, wherein if F, G, H and I are present, the ring fragment 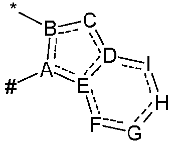 is represented by one of the following structures:

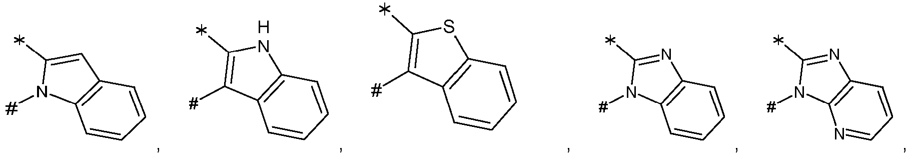

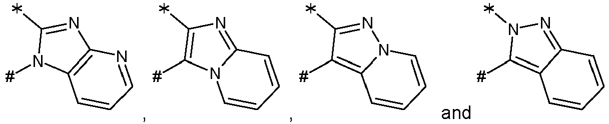

5. The compound according to any of claims 1 to 4, wherein
X is C(R^{a})R^{b};
*n is* 1; and
at least one of *m* and *p* is larger than 0.

6. The compound according to any of claims 1 to 5 selected from the group consisting of:

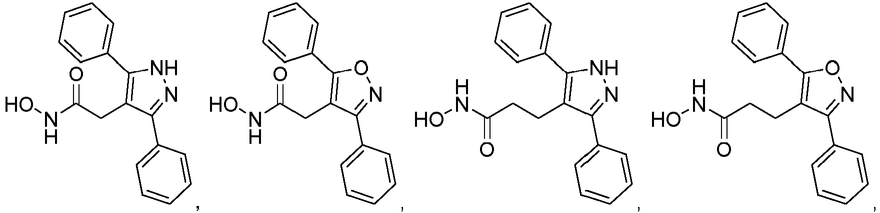

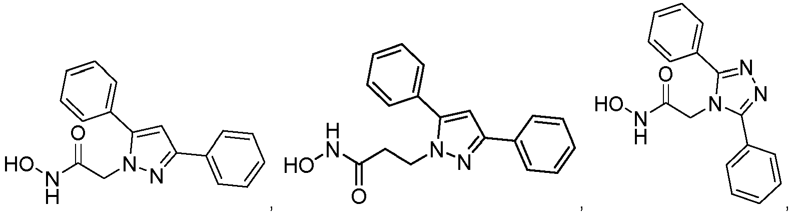

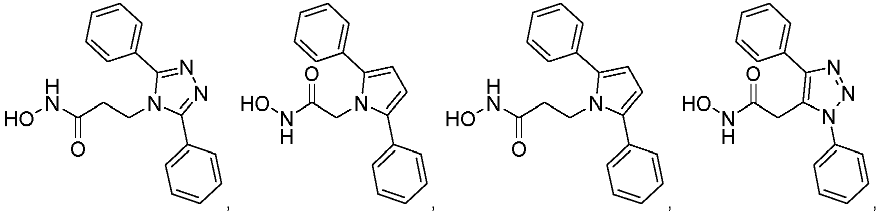

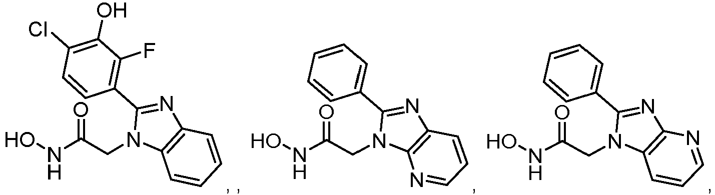

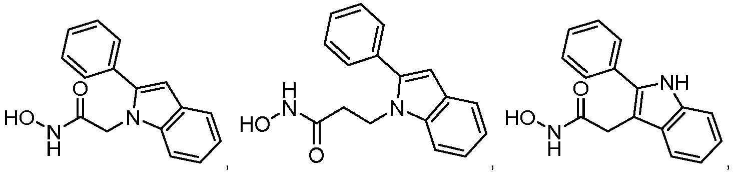

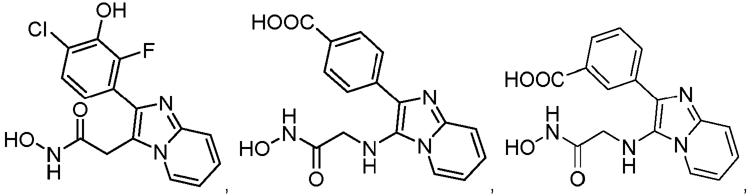

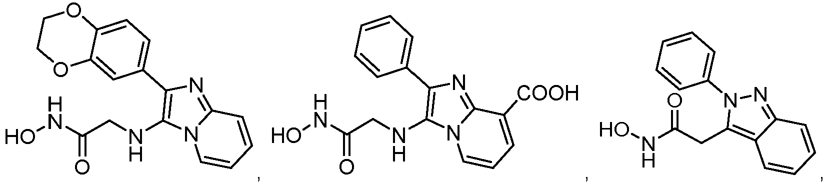

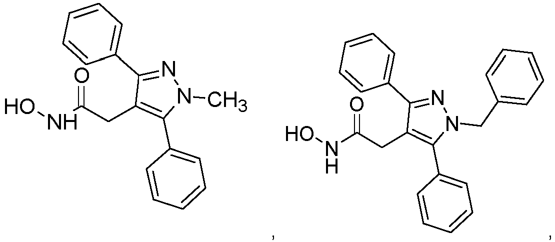

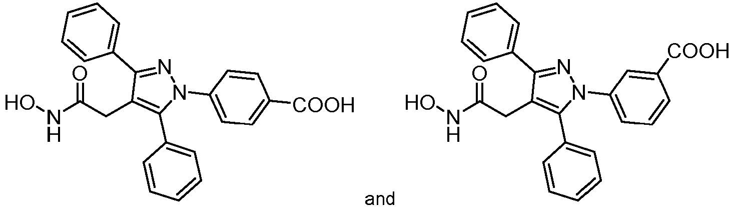

7. A pharmaceutical composition comprising the compound according to any of claims 1 to 6 and a pharmaceutically acceptable excipient.

8. A compound according to any of claims 1 to 7 or a pharmaceutical composition according to claim 9 for use in a method for treatment of the human or animal body.

9. A compound according to any one of claims 1 to 7 or a pharmaceutical composition comprising said compound according and a pharmaceutically acceptable excipient for use in a method for therapy or prevention of diseases and conditions selected from:
(a) Alzheimer's disease; nephritis; renal injury; renal ischemic injury; ischemic acute tubular necrosis; acute renal failure; bladder inflammation; inflammatory bowel disease (IBD); Crohn's disease; ulcerative colitis; chronic inflammation; colitis; fibrosis; fibrotic conditions; keloids; pulmonary hypertension; interstitial lung disease (ILD); cancer; colorectal cancer;
(b) fibrosis; acute fibrotic disorders and conditions; chronical fibrotic disorders and conditions; fibrosis occurring in organs and/or accompanying diseases and conditions selected from hepatitis, liver cirrhosis, hypertension, myocardial infarction, heart failure, asthma, pulmonary hypertension, scleroderma, fibrotic skin and internal organs, diabetes, diabetes nephropathy, atherosclerosis and fibrotic blood vessels; hypertrophic dermal scarring; keloids; pulmonary fibrosis; acute CNS scarring following traumatic injury; neuronal regeneration following stroke or spinal cord injury; obliterative fibrosis of the hollow structures within grafts; chronic allograft rejection; wound healing disorders; post-surgical scarring; dermal scarring; fibrosis resulting from gynecological procedures; fibrosis after eye surgery; fibrosis following angioplasty; fibrosis following surgery on joints; preventing local invasion, recurrence and metastasis of malignant keratinocytes or squamous cell carcinomas (SCCs);
(c) mammalian infertility; therapeutic use for *in vitro* fertilization (IVF) treatment of a mammal;
(d) nematode infections; infections caused by *Teladorsagia circumcincta*; infections caused by *Haemonchus contortus*; and infections caused by *Brugia malayi.*

## Patentansprüche

1. Verbindung gemäß der folgenden Formel I, 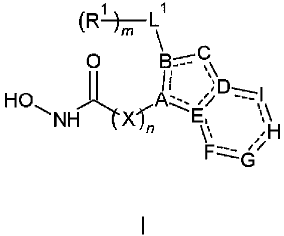 ihren einzelnen Enantiomeren, ihren einzelnen Diastereoisomeren, ihren Hydraten, ihren Solvaten, ihren Kristallformen, ihren einzelnen Tautomeren oder einem pharmazeutisch verträglichen Salz davon, wobei:
A unabhängig ausgewählt ist aus 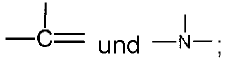
B unabhängig ausgewählt ist aus 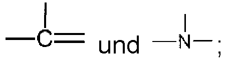
C unabhängig ausgewählt ist aus , 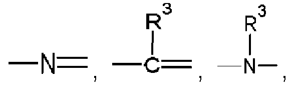 -O- und -S-;
F, falls vorhanden, unabhängig ausgewählt ist aus 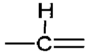 und -N=;
G, falls vorhanden, unabhängig ausgewählt ist aus 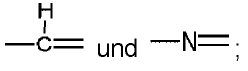
H, falls vorhanden, unabhängig ausgewählt ist aus 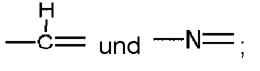
I, falls vorhanden, unabhängig ausgewählt ist aus 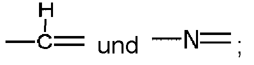
wobei, falls F, G, H und I vorhanden sind, dann:
ist D 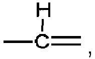
ist E unabhängig gewählt aus 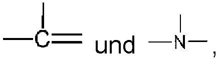 und
ist der Ring, der durch D, E, F, G, H und I ausgebildet ist, durch p Substituenten substituiert, dargestellt durch R², andernfalls, falls F, G, H und I fehlen, dann:
ist D unabhängig ausgewählt aus 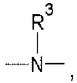 -N= und 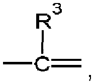 und
ist E unabhängig ausgewählt aus 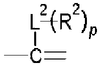 und -N=,
wobei jedes X unabhängig aus C(R^{a})R^{b} ausgewählt ist, wobei einer der C(R^{a})R^{b}-Gruppen durch eine NR^{a}-Gruppe ersetzt werden können;
*n* 1 oder 2 ist;
*m* 0, 1, 2 oder 3 ist;
*p 0,* 1, 2 oder 3 ist;
L¹ Phenyl ist;
L² Phenyl ist;
R¹ unabhängig ausgewählt ist aus Cl, F, OH, OCH₃ und COOH und/oder zwei R¹-Gruppen zusammen einen Teil eines 1,3-Benzodioxolrings oder eines 2,3-Dihydro-1,4-benzodioxinrings ausbilden;
R² COOH ist;
jedes R³ unabhängig ausgewählt ist aus Wasserstoff, Methyl, Ethyl, Propargyl, -CH₂COOH, Benzyl, unsubstituiertes Phenyl und substituiertes Phenyl, ausgewählt aus 3-Carboxyphenyl und 4-Carboxyphenyl; und
R^{a} and R^{b} Wasserstoff sind;
mit der Maßgabe, dass
wenn das Ringfragment 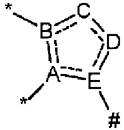 dargestellt ist durch 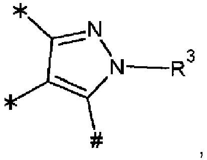 dann:
ist R³ ausgewählt aus Wasserstoff, Methyl, Benzyl und substituiertem Phenyl, ausgewählt aus 3-Carboxyphenyl und 4-Carboxyphenyl;
wenn das Ringfragment 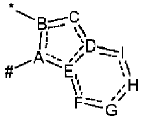 dargestellt ist durch 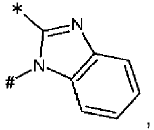 dann ist mindestens eines von *m* und *p* größer als 0; und
wenn das Ringfragment 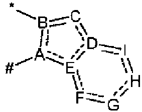 dargestellt ist durch 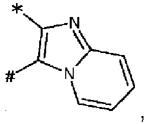 dann ist *m* größer als 0.

2. Verbindung nach Anspruch 1, die durch eine der folgenden Formeln la und Ib dargestellt wird:

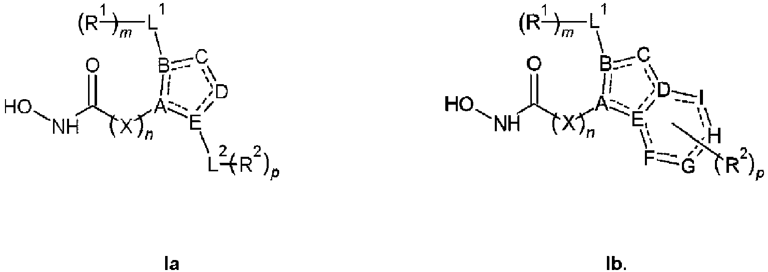

3. Verbindung nach einem der Ansprüche 1 oder 2, wobei, falls F, G, H und I fehlen, das Ringfragment 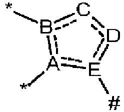 durch eine der folgenden Strukturen dargestellt wird: 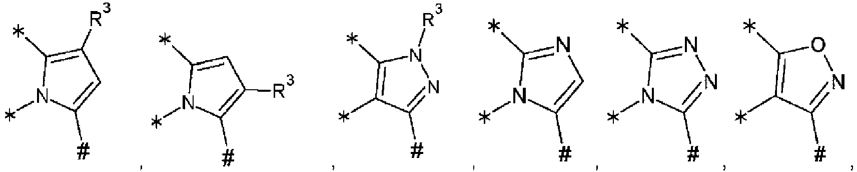 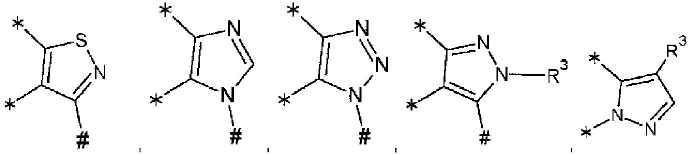 und

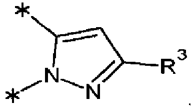

4. Verbindung nach einem der Ansprüche 1 bis 3, wobei, falls F, G, H und I vorhanden sind, das Ringfragment 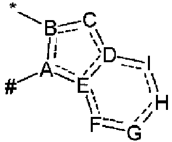 durch eine der folgenden Strukturen dargestellt wird:

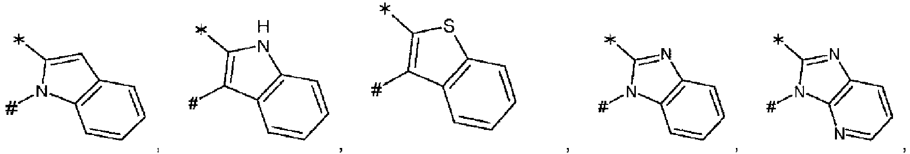

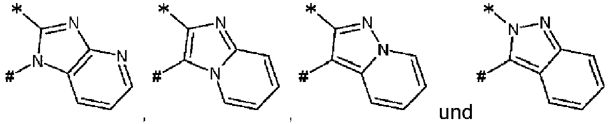

5. Verbindung nach einem der Ansprüche 1 bis 4, wobei
X C(R^{a})R^{b} ist;
*n* 1 ist; und
mindestens eines von *m* und *p* größer als 0 ist.

6. Verbindung nach einem der Ansprüche 1 bis 5, ausgewählt aus der Gruppe bestehend aus:

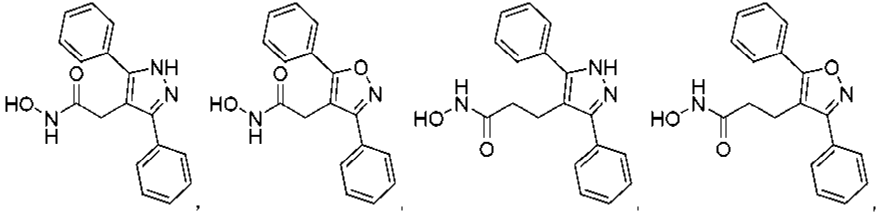

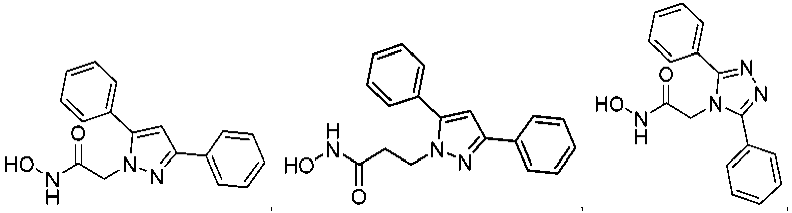

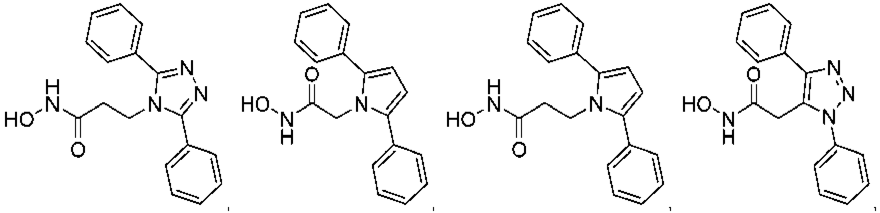

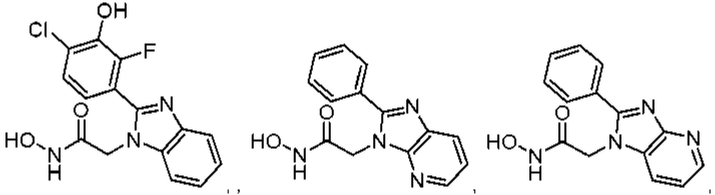

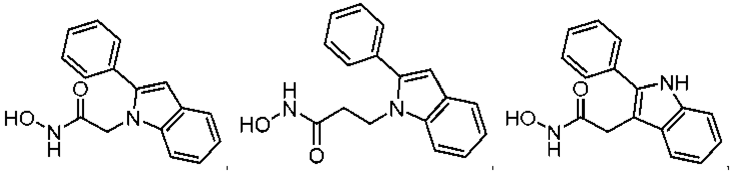

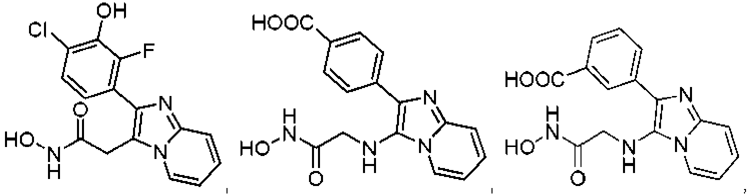

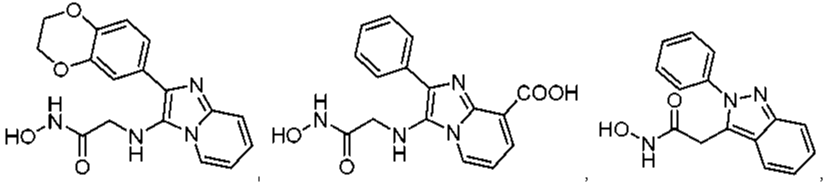

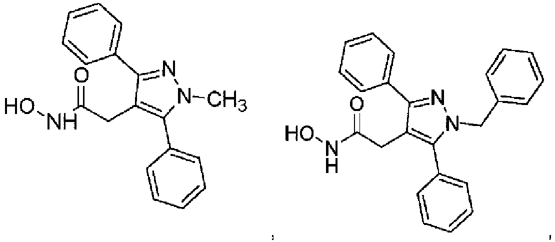

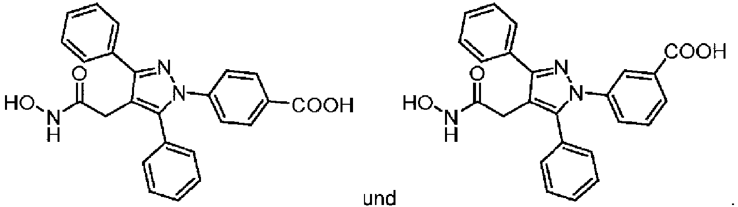

7. Pharmazeutische Zusammensetzung, umfassend die Verbindung nach einem der Ansprüche 1 bis 6 und einen pharmazeutisch verträglichen Träger umfasst.

8. Verbindung nach einem der Ansprüche 1 bis 7 oder eine pharmazeutische Zusammensetzung gemäß Anspruch 9 für eine Verwendung in einem Verfahren für eine Behandlung des menschlichen oder tierischen Körpers.

9. Verbindung nach einem der Ansprüche 1 bis 7 oder eine pharmazeutische Zusammensetzung, entsprechend umfassend die Verbindung und einen pharmazeutisch verträglichen Träger, für die Verwendung in einem Verfahren für eine Therapie oder Prävention von Krankheiten und Erkrankungen, ausgewählt aus:
(a) Alzheimer-Krankheit; Nephritis; Nierenschädigung; ischämische Nierenschädigung; ischämische akute Tubulusnekrose; akutes Nierenversagen; Blasenentzündung; entzündliche Darmerkrankung (IBD); Morbus Crohn; Colitis ulcerosa; chronische Entzündung; Kolitis; Fibrose; fibrotische Zustände; Keloide; pulmonale Hypertonie; interstitielle Lungenerkrankung (ILD); Krebs; Dickdarmkrebs;
(b) Fibrose; akute fibrotische Störungen und Erkrankungen; chronische fibrotische Störungen und Erkrankungen; in Organen auftretende Fibrose und/oder begleitende Krankheiten und Erkrankungen, ausgewählt aus Hepatitis, Leberzirrhose, Bluthochdruck, Herzinfarkt, Herzversagen, Asthma, pulmonaler Hypertonie, Sklerodermie, fibrotischer Haut und inneren Organen, Diabetes, diabetischer Nephropathie, Arteriosklerose und fibrotischen Blutgefäßen; hypertrophe dermale Narbenbildung; Keloide; Lungenfibrose; akute ZNS-Narbenbildung nach traumatischen Verletzungen; neuronale Regeneration nach Schlaganfall oder Rückenmarksverletzung; obliterative Fibrose der Hohlstrukturen innerhalb von Transplantaten; chronische Allotransplantatabstoßung; Wundheilungsstörungen; postoperative Narbenbildung; dermale Narbenbildung; Fibrose infolge gynäkologischer Eingriffe; Fibrose nach Augenoperation; Fibrose nach Angioplastie; Fibrose nach Gelenkoperationen; Verhinderung der lokalen Invasion, des Wiederauftretens und der Metastasierung maligner Keratinozyten oder Plattenepithelkarzinome (SCCs);
(c) Unfruchtbarkeit bei Säugetieren; therapeutische Verwendung für *in vitro* Befruchtungsbehandlung (IVF) eines Säugetiers;
(d) Nematodeninfektionen; Infektionen verursacht durch *Teladorsagia circumcincta;* Infektionen verursacht durch *Haemonchus contortus*; und Infektionen durch *Brugia malayi.*

## Revendications

1. Composé selon la formule I suivante, 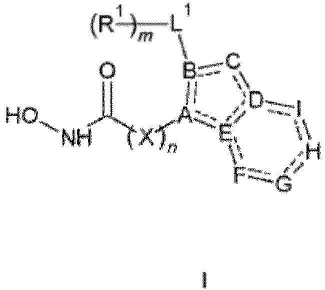 ses énantiomères individuels, ses diastéréo-isomères individuels, ses hydrates, ses solvates, ses formes cristallines, ses tautomères individuels ou un de ses sels pharmaceutiquement acceptables, où :
A est indépendamment choisi parmi 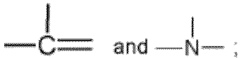
B est indépendamment choisi parmi 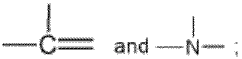
C est indépendamment choisi parmi -N=, 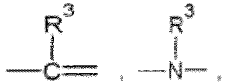 -O- and -S-,
F, s'il est présent, est choisi indépendamment parmi 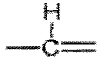 and -N=;
G, s'il est présent, est choisi indépendamment parmi 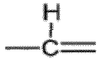 and -N=;
H, s'il est présent, est choisi indépendamment parmi 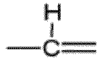 and -N=;
I, s'il est présent, est indépendamment choisi parmi 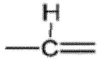 and -N=;
où si F, G, H et I sont présents, alors :
D est 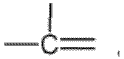
E est s élu indépendamment à partir du 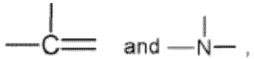 and
cycle formé par D, E, F, G, H et I est substitué par *p* des substituants représentés par R², sinon si F, G, H et I sont absents, alors :
D est indépendamment choisi parmi 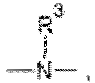 -N= and 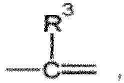 and
E est indépendamment choisi parmi 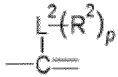 and -N**=**;
chaque X est indépendamment choisi parmi C(R^{a})R^{b}, où l'un des groupes C(R^{a})R^{b} pouvant être remplacé par un groupe NR^{a} ;
*n*est 1 ou 2 ;
*m* est 0, 1, 2 ou 3 ;
*p* est 0, 1, 2 ou 3 ;
L¹ est le phényle ;
L² est le phényle ;
R¹ est indépendamment choisi parmi CI, F, OH, OCH₃ et COOH, et/ou deux groupes R¹ font ensemble partie d'un cycle 1,3-benzodioxol ou d'un cycle 2,3-dihydro-1,4-benzodioxine ;
R² est COOH ;
chaque R³ est indépendamment choisi parmi l'hydrogène, le méthyle, l'éthyle, le propargyle, -CH₂COOH, le benzyle, le phényle non substitué et le phényle substitué choisi parmi le 3-carboxyphényle et le 4-carboxyphényle ; et
R^{a} et R^{b} sont l'hydrogène ;
sous réserve que
when the ring fragment 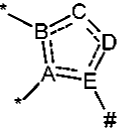 is represented by 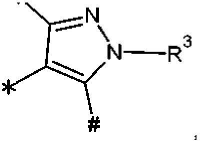 then:
R³ est choisi parmi l'hydrogène, le méthyle, le benzyle et le phényle substitué choisi parmi le 3-carboxyphényle et le 4-carboxyphényle ;
when the ring fragment 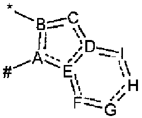 is represented by 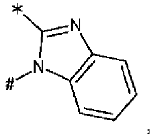 then at least one of m and *p* is larger than 0; and
when the ring fragment 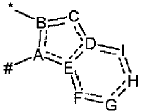 is represented by 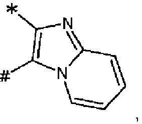 then *m* is larger than 0.

2. Composé selon la revendication 1, qui est représenté par l'une des formules la et Ib suivantes :

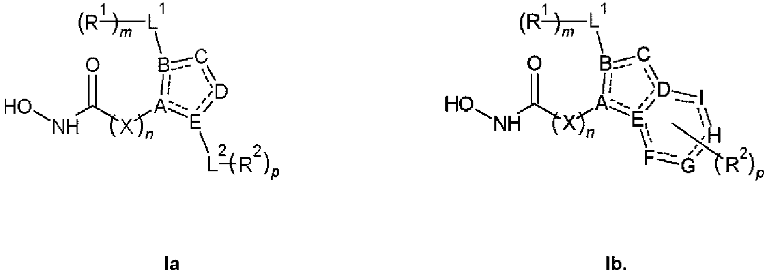

3. Composé selon l'une quelconque des revendications 1 ou 2, où, si F, G, H et I sont absents, le fragment de cycle est représenté par l'une des structures suivantes : 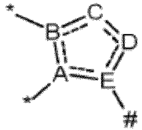 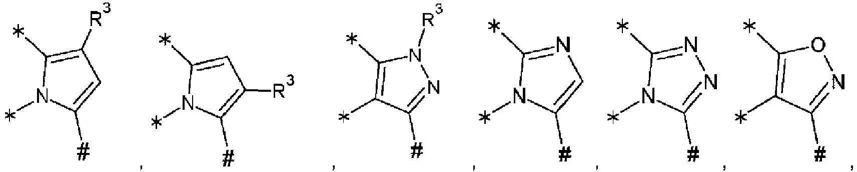 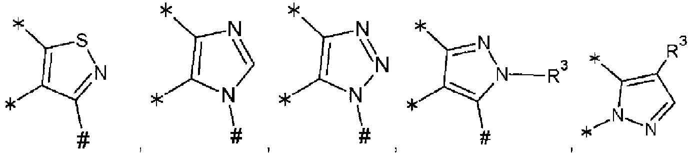 and

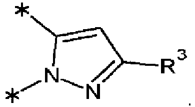

4. Composé selon l'une quelconque des revendications 1 à 3, où, si F, G, H et I sont présents, le fragment de cycle est représenté par l'une des structures suivantes :

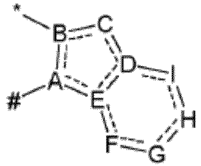

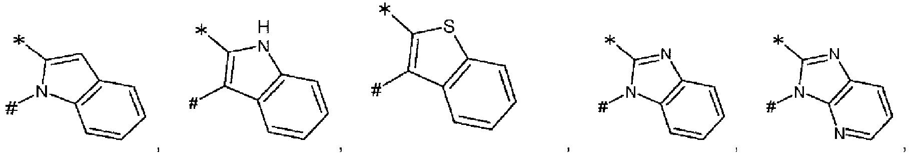

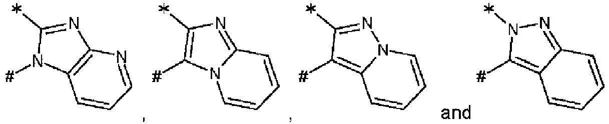

5. Composé selon l'une quelconque des revendications 1 à 4, dans lequel
X est C(R^{a})R^{b} ;
*n* est 1 ; et
au moins l'un parmi *m* et *p* est supérieur à 0.

6. Composé selon l'une quelconque des revendications 1 à 5, choisi dans le groupe constitué de :

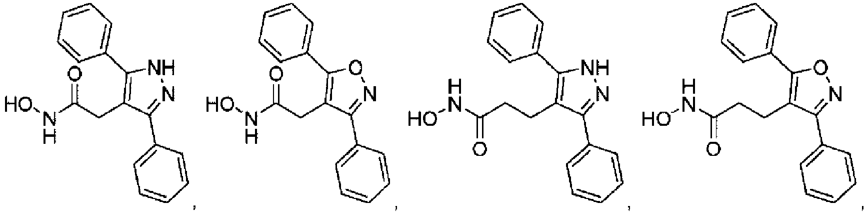

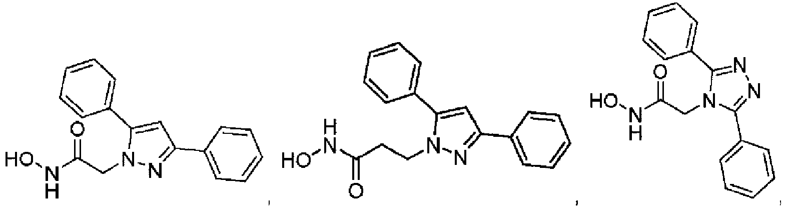

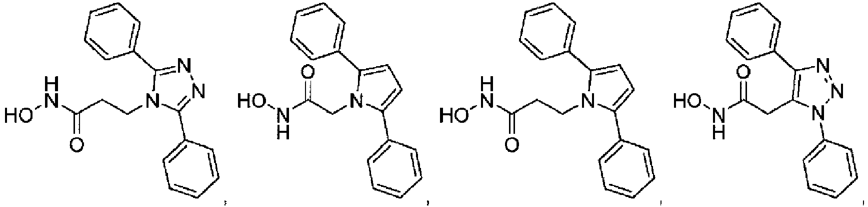

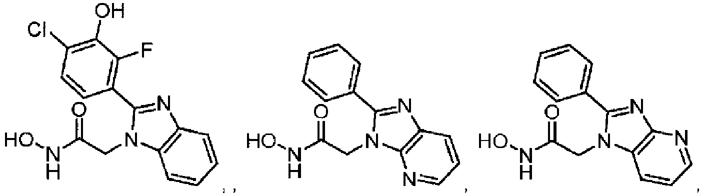

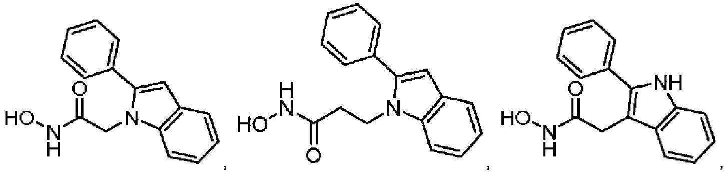

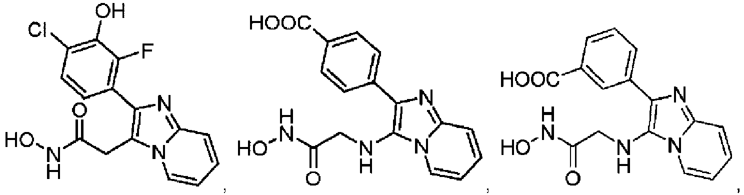

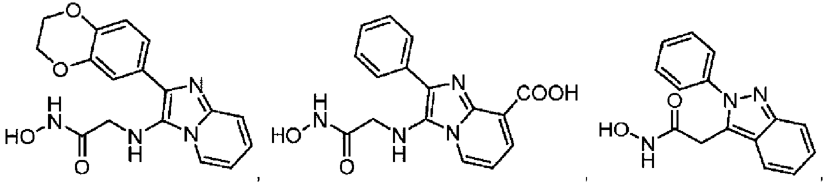

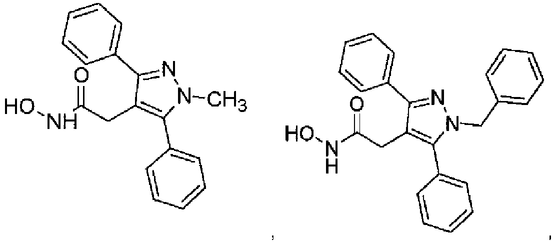

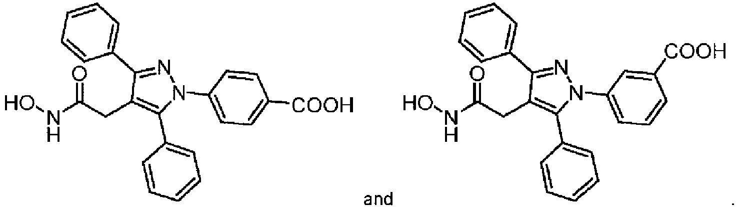

7. Composition pharmaceutique comprenant le composé selon l'une quelconque des revendications 1 à 6 et un excipient pharmaceutiquement acceptable.

8. Composé selon l'une quelconque des revendications 1 à 7 ou composition pharmaceutique selon la revendication 9 destiné à être utilisé dans un procédé de traitement du corps humain ou animal.

9. Composé selon l'une quelconque des revendications 1 à 7 ou composition pharmaceutique comprenant ledit composé et un excipient pharmaceutiquement acceptable destiné à être utilisé dans un procédé de thérapie ou de prévention de maladies et conditions sélectionnées parmi :
(a) la maladie d'Alzheimer ; la néphrite ; la lésion rénale ; la lésion ischémique rénale ; la nécrose tubulaire aiguë ischémique ; l'insuffisance rénale aiguë ; l'inflammation de la vessie ; la maladie inflammatoire chronique de l'intestin (MICI) ; la maladie de Crohn ; la colite ulcéreuse ; l'inflammation chronique ; la colite ; la fibrose ; les états fibrotiques ; les chéloïdes ; l'hypertension pulmonaire ; la maladie pulmonaire interstitielle (ILD) ; le cancer ; le cancer colorectal ;
(b) la fibrose ;; les troubles et affections fibrotiques aigus ; les troubles et affections fibrotiques chroniques ; la fibrose survenant dans des organes et/ou accompagnant des maladies et des états choisis parmi l'hépatite, la cirrhose du foie, l'hypertension, l'infarctus du myocarde, l'insuffisance cardiaque, l'asthme, l'hypertension pulmonaire, la sclérodermie, la fibrose cutanée et des organes internes, les diabètes, la néphropathie diabétique, l'athérosclérose et la fibrose des vaisseaux sanguins ; la cicatrisation dermique hypertrophique ; les chéloïdes ; la fibrose pulmonaire ; la cicatrisation aiguë du SNC à la suite d'une lésion traumatique ; la régénération neuronale à la suite d'un accident vasculaire cérébral ou une lésion de la moelle épinière ; la fibrose oblitérante des structures creuses à l'intérieur des greffons ; le rejet de greffe allogénique ; les troubles de cicatrisation de plaie ; la cicatrisation post-chirurgicale ; la cicatrisation dermique ; la fibrose résultant de procédures gynécologiques ; la fibrose à la suite d'une opération de l'œil ; la fibrose à la suite d'une angioplastie ; la fibrose à la suite d'une intervention chirurgicale sur les articulations ; la prévention d'une invasion locale, d'une récurrence et de métastases des kératinocytes malins ou des carcinomes squameux (CS) ;
(c) l'infertilité mammifère ; l'utilisation thérapeutique pour le traitement par fécondation *in vitro* (FIV) d'un mammifère ;
(d) les nématodoses ; les infections causées par *Teladorsagia circumcincta* ; les infections causées par *Haemonchus contortus* ; et les infections causées par *Brugia malayi.*
